# EUROPEAN PATENT APPLICATION

(11) **EP 1 382 615 A1**
(43) Date of publication of application: **21.01.2004**
(21) Application number: 02015591.7
(22) Date of filing: 15.07.2002
(51) Int. Cl.: C07K 14/705, C07K 16/28, C12N 15/12, A61K 39/395, A61K 48/00, A61P 35/00, G01N 33/53

(54) **MCAM inhibitors**

(71) Applicant: Xerion Pharmaceuticals AG, 82152 Martinsried (DE)
(72) Inventor: Unger, Christine Margarete, 82049 Grosshesselohe (DE); Zehetmeier, Carolin, 81541 München (DE)
(74) Representative: Fleuchaus, Andrea, Dr.

(57) **Abstract**

The present invention relates to polypeptides inhibiting the function of MCAM in naturally occuring tumor cells and their use, and the use of other molecules inhibiting MCAM function, for the treatment of specific cancers, particularly for reducing the invasiveness and/or the metastatic potential of specific cancer cells. Furthermore, a method is provided that allows to determine whether a naturally occurring tumor cell depends on functional MCAM for its invasiveness and/or metastatic potential. Finally, a method is provided that allows to identify an antibody or an antibody fragment capable of inhibiting invasiveness of sarcoma cells.

## Description

### BACKGROUND OF THE INVENTION

Malignant tumors shed cells, which migrate to new tissues and create secondary tumors. The process of generating secondary tumors is called metastasis and is a complex process in which tumor cells colonize sites distant from the primary tumor. Metastasis is a multi-step process in which tumor cells detach from the primary tumor, invade the cellular matrix, penetrate through blood vessels, thus enter the circulatory system (intravasation), arrest at a distant site, exit the blood stream (extravasation), and grow. (See, e.g., G. L. Nicolson (1982) Biochim. Biophis. Acta. 695, 113-176; G. L. Nicolson and G. Poste (1983) In. Rev. Exp. Pathol. 25, 77-181; G. Poste and I. J. Fidler (1980) Nature 283, 139-145; and E. Roos (1984) Biochim. Biophis. Acta. 738, 263). The process of metastasis of tumors has been proposed to involve cell adhesion molecules (CAM's), which mediate cell- cell or cell- matrix interactions.

A cell adhesion molecule which is up-regulated in various tumors is MCAM. MCAM is also known as MUC18, Mel-CAM or CD 146. MCAM is an integral membrane glycoprotein with an apparent molecular weight of M 113,000 Da. It contains five immunoglobulin-like domains, and its cytoplasmic domain contains several protein kinase recognition motifs, which suggests the involvement of MCAM in cell signaling (See, C. Sers et al. (1993) Proc. Nat. Sci. USA 90, 8514-8518).

Xie et al. (1997) Cancer Res. 57, 2295-2303 have shown that non-invasive SB-2 cells turn invasive after transfection with the MCAM cDNA. They have further shown that this artificially induced invasiveness can be inhibited with monoclonal antibodies against MCAM. However, it is dangerous and speculative to assign a physiological role for a protein based on a study in which it has been overexpressed, and it is an accepted standard in science to interpret results of overexpression studies with great care. To give only one example, the peroxisomal protein PEX 11 had been proposed, based on overexpression studies, to play a role in fatty acid oxidation. The careful studies of Li and Gould (2002) J. Cell Biol. 156(4):643-651 showed that PEX 11 had no such role and instead acts on peroxisome division.

The determination of the physiological role of a protein is a prerequisite for deciding whether interference with this protein's function might be a possible avenue for the treatment of disease or not. It must be kept in mind that in a physiological setting, that is to say in a naturally occurring tumor cell of a patient, MCAM is overexpressed together with other proteins which can modulate and change MCAM function. It is the functional interplay between MCAM and interacting proteins that determines MCAMs physiological role. Shih (1999) J. Pathol 189, 4-11 has demonstrated this by showing that the physiological role of MCAM relates to its interaction with its yet unidentified ligands. Importantly, the expression of MCAM-interacting proteins varies from tissue to tissue (Shih et al. (1997) Cancer Res. 57(17), 3835-3840) and for this reason the effects of MCAM overexpression must be seen in context. This is demonstrated by results obtained with another tumor type, i.e. breast cancer. Shih et al. (1997) Am. J. Pathol. 151(3), 745-751 revealed that in this context invasiveness was inhibited at high expression levels of MCAM. In this context MCAM even acted as a tumor suppressor. Therefore, the physiological role of MCAM in invasiveness and metastasis is still unclear.

The process of metastasis formation depends on the invasiveness of tumor cells. It would, therefore, be useful to develop drugs, which inhibit invasiveness and therewith prevent metastasis of primary tumors. The present invention demonstrates for the first time that for specific, naturally occurring tumor cells MCAM function is necessary for the process of invasion and/or metastasis.

### SUMMARY OF THE INVENTION

In an unbiased screen for molecules that can inhibit invasiveness of naturally occurring tumor cells surprisingly an antibody fragment binding to the extracellular domain of MCAM has been identified as such an inhibitor.

The present invention relates to polypeptides, which can specifically bind to the extracellular domain of MCAM and inhibit MCAM function. In preferred embodiments these polypeptides are antibody fragments or antibodies. Furthermore, the polypeptides of the invention can be labeled with detectable groups, if desired, or can be part of a bioconjugate.

The invention further relates to pharmaceutical compositions comprising a polypeptide of the invention.

In a further embodiment the invention relates to nucleic acid molecules encoding a polypeptide of the invention, as well as to vectors comprising such a nucleic acid and to host cells comprising such a vector.

In a further embodiment the invention relates to the use of molecules inhibiting MCAM function for the manufacture of a medicament for the treatment or prevention of invasion and/or metastasis of naturally occurring cancer cells, wherein invasiveness and/or metastatic potential of said cancer cells depends on MCAM function. In one embodiment such molecules are molecules, which inhibit gene expression of MCAM, like antisense oligonucleotides or interfering double stranded RNAs (siRNAs) or vectors that lead to the cellular production of such siRNAs. In another embodiment such molecules bind specifically to already expressed MCAM and inhibit MCAM function in invasion and/or metastasis. Such molecules are small chemical compounds or certain polypeptides binding to the extracellular region of MCAM, particularly a polypeptide or a bioconjugate of this invention.

In a further embodiment the invention relates to a method of treating or preventing invasion and/or metastasis in a patient, wherein the invasiveness and/or metastatic potential of said cancer cells depends on MCAM function.

In a further embodiment the invention relates to a method to determine the dependency of the invasiveness of a naturally occurring cancer cell on the functionality of MCAM.

In a further embodiment the invention relates to a method for the identification of antibody fragments useful for inhibiting the invasiveness of sarcoma cells, particularly the identification of such antibody fragments that bind to the extracellular domain of MCAM.

### DETAILED DESCRIPTION OF THE INVENTION

In order that the invention described herein may be more fully understood, the following detailed description is provided. As used herein, the following definitions shall apply unless otherwise indicated.

A "polypeptide" as used herein is a molecule comprising more than 10, preferably more than 20, most preferably more than 30, and less than 10000, more preferably less than 2500, most preferably less than 1000 aminoacids. Also polypeptides with substantial amino acid sequence identity and polypeptides which contain a low percentage of modified or non-natural amino acids are encompassed.

The terms " antibody" and "immunoglobulin", as used herein refer to any immunological binding agent, including polyclonal and monoclonal antibodies. Depending on the type of constant domain in the heavy chains, antibodies are assigned to one of five major classes: IgA, IgD, IgE, IgG, and IgM. Several of these are further divided into subclasses or isotypes, such as IgG1, IgG2, IgG3, IgG4, and the like. The heavy-chain constant domains that correspond to the different classes of immunoglobulins are termed alpha, delta, epsilon, gamma and mu, respectively. The subunit structures and three-dimensional configurations of different classes of immunoglobulins are well known.

Antibodies may be also selected from modified immunoglobulins, for example chemically or recombinantly produced antibodies, CDR grafted antibodies or humanized antibodies, site directed mutagenized antibodies that exhibit substantial amino acid sequence identity in their CDR regions, particularly in their CDR3 region, to the corresponding antibody fragments of the invention and retain substantially the same affinity for MCAM binding as the corresponding antibody fragments.

The CDRs (complementary determining region) of an antibody are the parts of these molecules that determine their specificity and make contact with specific ligands. The CDRs are the most variable parts of the molecule and contribute to the diversity of these molecules. They are structurally defined in a human IgG as amino acids 24 to 41 (CDR-L1), 50 to 57 (CDR-L2) and 90 to 101 (CDR-L3) of the light chain and amino acids 26 to 38 (CDR-H1), 51 to 70 (CDR-L2) and 100 to 125 (CDR-H3) of the heavy chain (see Kabat et al. (1987) 4th edn US Department of Health and Human Services, Public Health Service, NIH, Bethesda). The CDR regions of an antibody fragment can easily be determined by somebody skilled in the art by aligning the antibody fragment with said human IgG, e.g. using a program of the NCBI that allows to "Blast", and thereby align, two sequences with one another, and identifying the amino acids of the antibody fragment corresponding to the CDRs of a human IgG.

Substantial amino acid sequence identity as used herein means that at least 70%, preferably at least 85%, more preferably all but 5, still more preferably all but 3 and even more preferably all but 1 of the amino acids of two aligned amino acid sequences, particularly of aligned CDRs, are identical.

The term "antibody fragment" is used to refer to any fragment of an antibody-like molecule that has an antigen binding region, and this term includes antibody fragments such as scFv, dsFv, Fab', Fab, F(ab')₂, Fv, single domain antibodies (DABs), diabodies, and the like. The techniques for preparing and using various antibody-based constructs and fragments are well known in the art (see Kabat et al. (1991) J. Immunol. 147, 1709-19), specifically incorporated herein by reference.

"scFv" antibody fragments comprise the VH and VL domains of an antibody, wherein these domains are present in a single polypeptide chain. Generally, the scFv polypeptide further comprises a polypeptide linker between the VH and VL domains that enables the scFv to form the desired structure for antigen binding.

A "Fv" fragment is the smallest antibody fragment that retains an intact antigen binding site.

A "dsFv" is a disulfide stabilized Fv.

A "Fab" fragment, is an antigen binding fragment, containing complete light chains paired with the VH and CH1 domains of the heavy chain.

A "Fab"' fragment, is a reduced F(ab')₂ fragment.

A "F(ab')₂" fragment, is a bivalent fragment comprising two Fab fragments linked by a disulfide bridge at the hinge region.

A "single domain antibody (DAB)" is an antibody with only one (instead of two) protein chain derived from only one of the domains of the antibody structure. Dabs exploit the finding that, for some antibodies, half of the antibody molecule binds to its target antigen almost as well as the whole molecule (Davies et al. (1996) Protein Eng. 9: 531-537.

"Diabodies" are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (Hollinger et al. (1993) Proc. Natl. Acad. Sci. USA, 90: 6444-6448).

The terms "label" or "labeled" refers to a detectable marker or the incorporation of such, respectively, e.g., by incorporation of a fluorophore-, chromophore- or radio-labeled amino acid or attachment of a fluorophore-, chromophore- or radiolabel to a polypeptide or attachment of moieties that can be detected by a labeled second molecule containing a fluorescent marker or enzymatic activity that can be detected by an optical or a colorimetric method. An example for such a two-step detection system is the well known biotin-avidin system. Various methods of labeling polypeptides and glycoproteins are known in the art and may be used (for example see Lobl et al. (1988) Anal. Biochem., 170: 502-511).

An "epitope" includes any protein determinant capable of specific binding to an immunoglobulin or an antibody fragment. Epitopic determinants usually consist of chemically active surface groupings of molecules such as exposed amino acids, aminosugars, or other carbohydrate side chains and usually have specific three-dimensional structural characteristics, as well as specific charge characteristics.

A "naturally occurring cancer cell" as used herein is a cell that has not been transfected, transduced or otherwise genetically engineered in the laboratory. Such a cell does not comprise artificial DNA sequences, e.g. of vectors, or DNA sequences being found only in other species, but not usually in the species from which the naturally occurring cancer cell was derived. However, a naturally occurring cancer cell may comprise sequences that are not usually found in the species from which it was derived, if those sequences have arisen due to the processes of mutation and selection that took place within the individuum from which the naturally occurring cancer cell was derived, and/or during continued culture of the naturally occurring cancer cell.

Selected cancer cell-types as used herein consist of astrocytoma, fibrosarcoma, myxosarcoma, liposarcoma, oligodendroglioma, ependymoma, medulloblastoma, primitive neural ectodermal tumor (PNET), chondrosarcoma, osteogenic sarcoma, pancreatic ductal adenocarcinoma, small and large cell lung adenocarcinomas, chordoma, angiosarcoma, endotheliosarcoma, squamous cell carcinoma, bronchoalveolarcarcinoma, epithelial adenocarcinoma, and liver metastases thereof, lymphangiosarcoma, lymphangioendotheliosarcoma, hepatoma, cholangiocarcinoma, synovioma, mesothelioma, Ewing's tumor, rhabdomyosarcoma, coloncarcinoma, basal cell carcinoma, sweat gland carcinoma, papillary carcinoma, sebaceousgland carcinoma, papillary adenocarcinoma, cystadenocarcinoma, medullary carcinoma, bronchogenic carcinoma, renal cell carcinoma, bileduct carcinoma, choriocarcinoma, seminoma, embryonal carcinoma, Wilms' tumor, testicular tumor, medulloblastoma, craniopharyngiorna, ependymoma, pinealoma, hemangioblastoma, acoustic neurorna, oligodendroglioma, meningioma, neuroblastorna, retinoblastoma, leukemia, multiplemyelorna, Waldenstrom's macroglobulinemia, and heavy and light chain disease, and adenocarcinomas of the uterine cervix, uterine and ovarian epithelial carcinomas, transitional, cell carcinoma of the bladder, B and T cell lymphomas (nodular and diffuse) plasmacytoma, acute and chronic leukemias, soft tissue sarcomas and leiomyosarcomas.

"Treating metastatic tumors", as used herein means that the metastasis of the tumor is prevented, delayed, or inhibited.

"Metastatic tumors" as used herein include both tumors at the primary site capable of metastasizing and metastasized tumors at a secondary site. Such metastatic tumors can be of a tissue origin of the lung, liver, kidney, stomach, small intestine, bone, spleen, brain, peripheral nervous system, thyroid, pancreatic, endometrial, ovarian, cervical, skin, colon or lymphoid tissue.

"Invasiveness" as used herein is the ability of a cell to migrate through a layer of other cells or to migrate through the extracellular matrix. Invasiveness can be assessed by the Matrigel assay described in Example 5. Invasion is measured as cells that reach the lower surface of the filter during a certain incubation period. When more than 40% of cells within 6h to 12h reach the other side of the filter and form colonies in an invasion assay like in Example 5, the naturally occurring cancer cell is defined as invasive. The control cells instead form only 5% colonies in the same time frame and are defined as non-invasive.

"Adhesiveness" as used herein is the ability of a cell to reattach after they have been removed from the matrix on which it had been grown, resuspended as a solution of single cells (not in direct contact with other cells of the solution), and replated on a matrix to which adhesion is possible. A cell is defined as adhesive if in an assay as described in Example 7, more than 40% of the cells adhere within a time of 30-120 min. Instead, only 5% of the control cells adhere within the same time frame.

Metatstatic potential as used herein is the ability of a tumor cell to form a new tumor at a site distant from the primary tumor of which the tumor cell was derived (a metastase). Metastatic potential can be measured by injecting, e.g. 1×10⁶ , cells into the lateral tail vein of athymic nude mice and determining the number of tumor nodules in the lung, e.g. 2 months post injection, e.g. as described in the section "Tumor cell injections" on page 2346 of Huang et al (1996) Oncogene 13: 2339-2347, or the sections "Animals and production of tumors" and " Histochemical analysis for calcified matrix" on page 1882 of Radinsky et al. (1994) Oncogene 9: 1877-1883. A cell line to produce more than 3, preferably more than 8, more preferably more than 20 tumor nodules in the lung in this assay is considered metastatic.

Therapeutically effective amounts are amounts which eliminate or reduce the patient's tumor burden, or which prevent or reduce metastasis. The dosage will depend on many parameters, including the nature of the tumor, patient history, patient condition, the possible co-use of cytotoxic agents, and methods of administration. Methods of administration include injection (e.g., parenteral, subcutaneous, intravenous, intraperitoreal, etc), for which the molecule inhibiting MCAM function is provided in a nontoxic pharmaceutically acceptable carrier. In general, suitable carriers and diluents are selected so as not to significantly impair biological activity of the binding agent (e.g., binding specificity, affinity or stability), such as water, saline, Ringer's solution, dextrose solution, 5% human serum albumin, fixed oils, ethyloleate, or liposomes.). Acceptable carriers can include biocompatible, inert or bio-absorbable salts, buffering agents, oligo- or polysaccharides, polymers, viscoelastic compound such as hyaluronic acid, viscosity-improving agents, preservatives, and the like. In addition, the pharmaceutical composition or formulation may also include other carriers, adjuvants, or nontoxic, non-therapeutic, non-immunogenic stabilizers and the like. Typical dosages may range from about 0.01 to about 20 mg/kg, or more particularly from about 1 to about 10 mg/kg.

Therapeutic methods employing molecules inhibiting MCAM function may be combined with chemotherapy, surgery, and radiation therapy, depending on type of the tumor, patient condition, other health issues, and a variety of factors.

A "molecule inhibiting MCAM function", is a molecule resulting in inhibition of the biological activity of MCAM. This inhibition of the biological activity of MCAM can be assessed by measuring one or more indicators of MCAM's biological activity, such as MCAM dependent invasiveness or MCAM dependent adhesion. These indicators of MCAM's biological activity can be assessed by one or more of several *in vitro* or *in vivo* assays (see, Examples 5 and 7). Preferably, the ability of a molecule to inhibit MCAM activity is assessed by inhibition of MCAM-induced invasiveness or adhesion of invasive human sarcoma cells, particularly the cells used in Examples 5 and 7.

A "molecule inhibiting MCAM function" of the invention is not a molecule which is a general inhibitor of protein function, like a protease, like a denaturing agent, e.g.urea or guanidinium hydrochloride, like heavy metal atoms or like small molecules (e.g. aldehyds or isocyanates) reacting covalently and non-specifically with biomolecules (lipids, proteins, sugars). A molecule inhibiting MCAM function is characterized by its ability to inhibit MCAM function at a concentration at which it does not inhibit the function of the insulin receptor (e.g. as determined in an anti-Phosphotyrosine Western Blot Assay, see e.g. B. Cariou et al. (2002) J Biol Chem., 277, 4845-52) and the Acetylcholin receptor (e.g. as determined by measuring the Ca influx, see M. Montiel et al. (2001) Biochem Pharmacol. 63, 337-42.) and the B-CAM cell surface glycoprotein (e.g. by determining binding of hemoglobin A red blood cells (AA RBCs) to immobilized laminin as described in the section "Flow chamber assays" on page 2551 of Udani et al. (1998) J. Clin. Invest. 101(11): 2550-2558). Only a molecule inhibiting MCAM function but at the same concentration not significantly affecting the function of the other three receptors mentioned is a molecule inhibiting MCAM function as used in this patent. Inhibition is understood to be at least a 30%, preferably a 40%, more preferably a 50%, even more preferably a 60% decrease in function, as defined by MCAM function in an invasion assay as mentioned above, when compared to a negative control with the same experimental conditions, but without the molecule of the invention. A molecule is defined as not significantly affecting the function of the other three receptors if the decrease in function affected by the molecule of the invention is less than 20%, more preferably less than 15%, even more preferably less than 10%.

Additionally, in the case of a molecule of the invention which inhibits gene expression of MCAM, such a molecule decreases MCAM expression by more than 50%, preferably by more than 80%, still more preferably by more than 90%, most preferably by more than 95% when measured in a quantitative western blot normalized to the level of beta tubulin present, when present in an experiment at a concentration of 10 nM to 100 µM, preferably at around 1 µM, in which the amount of MCAM is compared between two otherwise identical samples, wherein in one sample the molecule of the invention was allowed to inhibit MCAM expression. In the same experiment the molecule of the invention does not decrease the amount of the beta tubulin present per cell by more than 20%, and said molecule does not decrease the relative level of the insulin receptor and the B-CAM cell surface glycoprotein by more than 20%.

Additionally, in the case of a polypeptide of the invention, particularly an antibody or antibody fragment of the invention, the polypeptide of the invention is considered to inhibit the biological function of MCAM if it reduces the invasiveness of naturally invasive cancer cells in an experiment as in Example 5 or 7 by more than 30%, preferably more than 60%, when said antibody fragment is present at a concentration of 1 nM to 50 µM, preferably around 20 µM.

Additionally, in the case of a small chemical compound of the invention, said compound is considered to inhibit the biological function of MCAM if it reduces the invasiveness of naturally invasive cancer cells in an experiment as in Example 5 or 7 by more than 30%, preferably by more than 60%, when present at a concentration of 10 nM to 100 µM, preferably at around 1 µM, while not affecting cell morphology, cell cycle progression (determined by analyzing the DNA content of a cell population by propidium iodide staining and FACS analysis), and not increasing the percentage of the cells of the culture that show signs of apoptosis (determined by measuring the percentage of cells showing DNA fragmentation, e.g. by a so called tunnel-assay). A small chemical compound as used in this invention is a molecule with a molecular weight between 50 Da and 10000 Da, preferably between 100 Da and 4000 Da, more preferably between 150 Da and 2000 Da, or a physiologically acceptable salt thereof.

A molecule "binding specifically to MCAM" or "specific for MCAM" as mentioned herein is a molecule which binds to MCAM expressing HT 1080 cells, but not to Hs-27 cells or MCAM-negative SBcl-2 cells (Shih et al. (1997) Cancer Res. 57(17), 3835-3840) under the conditions given in Examples 1 and 2. That is to say that binding to HT 1080 cells is at least 2 fold higher, preferably 5 fold higher and most preferably 20 fold higher than binding to Hs-27 cells or SBcl-2 cells when said molecule is tested at a concentration of 0,1 nM to 10 µM, preferably 1 nM to 1 µM, still more preferably 10 nM to 500 nM and most preferably around 100 nM.

Such a molecule may additionally have a binding constant for MCAM lower than 10 µM, preferably lower than 1 µM, more preferably lower than 100 nM, most preferably from 0,1nM to 20 nM. Binding constants can be determined, e.g., by standard methods like the BIACORE system according to the instructions of the manufacturer's manual.

The term "at least one" as used here means "one and more than one", particularly one, two, three, four and five.

The present invention relates to polypeptides, which can specifically bind to the extracellular region of MCAM and inhibit MCAM function in invasion and/or metastasis. Those polypeptides comprise a sequence selected from the group consisting of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8 and SEQ ID NO. 9, particularly selected from the group consisting of SEQ ID NO. 2, SEQ ID NO. 3, SEQ ID NO. 4, SEQ ID NO. 5, SEQ ID NO. 6, SEQ ID NO. 7, SEQ ID NO. 8 and SEQ ID NO. 9.

In a preferred embodiment the polypeptide of the invention is an antibody fragment, in particular a scFv, dsFv, Fab', Fab, F(ab')2, Fv, single domain antibody or diabody, more particularly a scFv, dsFv, Fv, single domain antibody or diabody, still more particularly a scFv, single domain antibody or diabody and even more preferably a scFv.

In another preferred embodiment the polypeptide of the invention is an antibody, in one preferred embodiment an antibody derived from a scFv antibody fragment, in another preferred embodiment a polyclonal or monoclonal antibody, particularly a human monoclonal antibody.

Anti-human MCAM binding antibodies may be selected from modified immunoglobulins, for example chemically or recombinantly produced antibodies or humanized antibodies, site directed mutagenized antibodies, that exhibit substantial amino acid sequence identity in their CDR regions, particularly in their CDR3 region, to the corresponding antibody fragments of the invention and retain substantially the same affinity for MCAM binding as the corresponding antibody fragments.

In another preferred embodiment the polypeptide of the invention is a human antibody selected from the group consisting of IgA, IgD, IgE, IgG, and IgM, in particular IgG and IgM, more particularly IgG1, IgG2a, IgG2b, IgG3, IgG4.

In another preferred embodiment the CRD3 region of the antibody or the antibody fragment is identical to one of the CDR3 regions shown in Table 1.

In another preferred embodiment of the invention a polypeptide of the invention, particularly an antibody fragment or an antibody of the invention, is labeled with a detectable label. Particularly, examples for detectable labels are radioisotopes, chromophores, fluorophores, enzymes or radioisotopes. The detectable label can, for example, be selected from this group.

In another embodiment, the polypeptide of the invention can be covalently or non-covalently conjugated and/or coupled to or with, respectively, another protein, a solid matrix (e.g. like a bead), with itself to form multimers, a cytotoxic agent further enhancing the toxicity to targeted cells, a cytostatic agent, a prodrug, or an effector molecule, which is able to modify the cell expressing MCAM or to recruit immune cells. All these conjugates are "bioconjugates" of the invention.

A list of cytotoxic agents include, but is not limited to, daunorubicin, taxol, adriamycin, methotrexate, 5 FU, vinblastin, actinomycin D, etoposide, cisplatin, doxorubicin, genistein, andribosome inhibitors (e.g., trichosantin), or various bacterialtoxins (e.g., Pseudomonas exotoxin; Staphylococcus aureus protein A).

Bioconjugates comprising the polypeptides of the invention, particularly the antibody fragment or antibody of the invention, together with said cytotoxic moieties are made using a variety of bifunctional protein coupling agents. Some examples of such reagents are N-succinimidyl 3-(2-pyridyldithio)-propionate (SPDP), bifunctional derivatives of imidoesters such a dimethyl adipimidate HCl, active esters such as disuccinimidyl suberate, aldehydes such as glutaraldehyde, bisazido compounds such as his (R-azidobenzoyl) hexanediamine, bisdiazonium derivatives such as bis-(R-diazoniumbenzoyl)ethylenediamine, diisocyanates such as tolylene 2,6-diisocyanate, and bis-activated fluorine compounds such as 1,5-difluoro-2,4-dinitrobenzene. Methods useful for the production of bioconjugates are described in detail in March's Advanced Organic Chemistry: Reactions, Mechanisms and Structure, 5th Edition, Wiley-Interscience; or Bioconjugate Techniques, Ed. Greg Hermanson, Academic Press.

The expression of a metastasis-associated MCAM antigen can be detected by using a bioconjugate or a polypeptide of the invention, particularly an antibody or an antibody fragment of the invention. A sample is taken from the subject, e.g., a biopsy specimen taken from tissue suspected of having a metastatic tumor. Generally, the sample is treated before an assay is performed. Assays, which can be employed include ELISA, RIA, EIA, Western Blot analysis, immunohistological staining and the like. Depending upon the assay used, the antigens or the antibodies can be labeled by an enzyme, a fluorophore or a radioisotope. (See, e.g., Coligan et al. (1994) Current Protocols in Immunology, John Wiley & Sons Inc., New York, New York; and Frye et al. (1987) Oncogene 4: 1153-1157.)

Therefore, one embodiment of the invention relates to the use of at least one polypeptide of the invention and/or at least one labeled polypeptide of the invention and/or at least one bioconjugate of the invention for the detection of MCAM. For example one polypeptide of the invention or one labeled polypeptide of the invention or one bioconjugate of the invention can be used for the detection of MCAM, or one labeled polypeptide together with one bioconjugate or two or three polypeptides or two labeled polypeptides can be used.

In another embodiment, the present invention encompasses a diagnostic kit. Such a kit comprises at least one bioconjugate and/or at least one labeled polypeptide of the invention and/or at least one polypeptide of the invention, particularly an antibody fragment or an antibody of the invention, or a labeled version of these, and consists additionally of the reagents and materials necessary to carry out a standard competition or sandwich assay. Said diagnostic kit may be used for the determination of the invasive potential of biological samples, in particular of certain cancer cell types. A kit will further typically comprise a container.

By using the polypeptide of the invention, particularly the antibody fragment or antibody of the invention, it is further possible to produce anti-idiotypic antibodies, which can be used to screen antibodies to identify whether the antibody has the same binding specificity as a human monoclonal antibody of the invention and can also be used for active immunization (Herlyn et al. (1986) Science, 232:100). Such anti-idiotypic antibodies can be produced using well-known hybridoma techniques (Kohler et al. (1975) Nature, 256:495). An anti-idiotypic antibody is an antibody, which recognizes unique determinants present on the antibody of interest. These determinants are located in the hypervariable region of the antibody. It is this region, which binds to a given epitope and, thus, is responsible for the specificity of the antibody. An anti-idiotypic antibody can be prepared by immunizing an animal with the polypeptide, particularly the antibody fragment or antibody, of interest. The immunized animal will recognize and respond to the idiotypic determinants of the immunizing antibody and produce an antibody to these idiotypic determinants. By using anti-idiotypic antibodies, it is possible to identify other hybridomas expressing monoclonal antibodies having the same epitopic specificity.

It is also possible to use the anti-idiotype technology to produce monoclonal antibodies, which mimic an epitope. For example, an anti-idiotypic monoclonal antibody made to a first monoclonal antibody will have a binding domain in the hypervariable region, which is the "image" of the epitope bound by the first antibody. Thus, the anti-idiotypic monoclonal antibody can be used for immunization, since the anti-idiotype monoclonal antibody binding domain effectively acts as an antigen.

In another embodiment the polypeptide of the invention, particularly the antibody fragment or antibody of the invention, binds to human MCAM and reduces the invasiveness of invasive human sarcoma cells by 30-60%, or preferably by 30-55%, 40-50% or even at least 60%, when tested in an invasion assay (see example 5).

In another embodiment, the antibody fragment of the invention specifically recognizes one or more epitopes of MCAM, or epitopes of conserved variants of MCAM, or peptide fragments of the MCAM.

In another embodiment, the invention relates to the use of a molecule of the invention, particularly selected from the group consisting of a small chemical compound of the invention, a molecule inhibiting gene expression of MCAM, a bioconjugate or a polypeptide of the invention, more particularly an antibody fragment or an antibody of the invention as a medicament.

In another embodiment, the present invention relates to a pharmaceutical composition comprising effective amounts of at least one, particularly one, molecule of the invention, particularly at least one of any of the polypeptides of the invention or at least one of a molecule inhibiting gene expression of MCAM, more particularly wherein the molecule is an antisense oligonucleotide against MCAM, an interfering dsRNA (siRNA) or siRNA-like hairpin RNA against MCAM or a vector leading to cellular presence of siRNA against MCAM or siRNA-like hairpin RNA against MCAM, or alternatively wherein the molecule is an antibody fragment or antibody of the invention, in combination with a pharmaceutically acceptable carrier and/or a diluent. The pharmaceutical composition can be used for the treatment of conditions related to the over-expression or ectopic expression of human MCAM, especially the treatment of metastatic tumors, especially of metastatic tumors derived from the group selected of cancer cell-types of pages 7 and 8.

In another embodiment of the invention, pharmaceutical compositions are provided comprising a pharmaceutically acceptable carrier and a therapeutically effective amount of at least one molecule inhibiting MCAM function, particularly wherein the molecule inhibits gene expression of MCAM, more particularly wherein the molecule is an antisense oligonucleotide against MCAM, an interfering dsRNA (siRNA) or siRNA-like hairpin RNA against MCAM or a vector leading to cellular presence of siRNA against MCAM or siRNA-like hairpin RNA against MCAM. Alternatively the molecule inhibiting MCAM function can particularly be a molecule which can bind to the extracellular region of MCAM, more particularly wherein the molecule is a small chemical compound or a polypeptide or the bioconjugate of the invention, still more particularly wherein the molecule is an antibody fragment of the invention, even more preferably wherein the molecule is a scFv of the invention or an antibody derived from such a scFv of the invention.

In another embodiment, the invention relates to administering a molecule inhibiting MCAM function in a pharmaceutical acceptable composition, particularly wherein the molecule inhibits gene expression of MCAM, more particularly wherein the molecule is an antisense oligonucleotide against MCAM, an interfering dsRNA (siRNA) or siRNA-like hairpin RNA against MCAM or a vector leading to cellular presence of siRNA against MCAM or siRNA-like hairpin RNA against MCAM. Alternatively the molecule inhibiting MCAM function can particularly be a molecule which can bind to the extracellular region of MCAM, more particularly wherein the molecule is a small chemical compound or an antibody or an antibody fragment or a polypeptide of the invention or a bioconjugate of the invention, still more particularly wherein the molecule is an antibody fragment of the invention, even more preferably wherein the molecule is a scFv of the invention or an antibody derived from such a scFv of the invention.

The present invention further relates to a method to produce the polypeptide of the invention by recombinant techniques. These techniques are well known in the art (Skerra et al. (1993), Curr. Opin. Immunol. 5, 256-62; Chadd et al. (2001), Curr. Opin. Biotechnol. 12, 188-94).

For example, nucleic acid sequences encoding a polypeptide of the invention, particularly an antibody fragment or an antibody (e.g., a gene encoding an antibody fragment of Tables 1 or 2 or an antibody thereof) can be isolated and cloned into one or more polynucleotide expression vectors, and the vector can be transformed into a suitable host cell line for expression of a recombinant polypeptide of the invention. Expression of the gene encoding the polypeptide of the invention provides for increased yield of the polypeptide, and also allows for routine modification of the polypeptide by introducing amino acid substitutions, deletions, additions and other modifications, for example humanizing modifications (Rapley (1995) Mol. Biotechnol. 3: 139-154) in both the variable and constant regions of the antibody fragment of the antibody of the invention without critical loss of binding specificity or MCAM blocking function (Skerra et al. (1993) Curr. Opin. Immunol. 5; 256-262).

The present invention therefore relates to an above mentioned isolated nucleic acid molecule encoding any one of the polypeptides of the invention, particularly an antibody fragment of the invention, more particularly a scFv, dsFv, Fv, single domain antibody or diabody of the invention, still more particularly a scFv, single domain antibody, diabody of the invention or an antibody derived from such a scFv of the invention, and even more preferably a scFv of the invention or an antibody derived from such a scFv of the invention.

In a preferred embodiment the present invention relates to a nucleic acid molecule encoding a polypeptide comprising a sequence selected from the group consisting of SEQ ID NO. 1, SEQ ID NO. 2, SEQ ID NO. 3, SEQ ID NO. 4, SEQ ID NO. 5, SEQ ID NO. 6, SEQ ID NO. 7, SEQ ID NO. 8, SEQ ID NO. 9, as shown in Table 1, particularly selected from the group consisting of SEQ ID NO. 2, SEQ ID NO. 3, SEQ ID NO. 4, SEQ ID NO. 5, SEQ ID NO. 6, SEQ ID NO. 7, SEQ ID NO. 8 and SEQ ID NO. 9. More particularly the isolated nucleic acid sequence of the invention comprises any one of the nucleic acid sequences selected from the group consisting of SEQ ID NO. 10, SEQ ID NO. 11, SEQ ID NO. 12, SEQ ID NO. 13, SEQ ID NO. 14, SEQ ID NO. 15, SEQ ID NO. 16, SEQ ID NO. 17 and SEQ ID NO. 18, even more particularly selected from the group consisting of SEQ ID NO. 11, SEQ ID NO. 12, SEQ ID NO. 13, SEQ ID NO. 14, SEQ ID NO. 15, SEQ ID NO. 16, SEQ ID NO. 17 and SEQ ID NO. 18.

The present invention further relates to a vector comprising a nucleic acid of the invention. Particularly the vector is a plasmid, a phagemid, or a cosmid.

For example, the nucleic acid molecule of the invention can be cloned in a suitable fashion into procaryotic or eucaryotic expression vectors (Sambrook et al., "Molecular cloning: a laboratory manual" Second edition, Cold Spring Harbor Laboratory Press (1989)). Such expression vectors comprise at least one promotor, at least one signal for translation initiation, at least one nucleic acid sequence of the invention and - in the case of procaryotic expression vectors - a signal for translation termination, while in the case of eucaryotic expression vectors preferably additional signals for transcriptional termination and for polyadenylation.

Examples for prokaryotic expression vectors are, for expression in *Escherichia coli,* e.g. expression vectors based on promotors recognized by T7 RNA polymerase, as described in US 4,952,496, for eucaryotic expression vectors for expression in Saccharomuces serevisiae , e.g., the vectors p426Met25 or 526GAL1 (Mummberg et al. (1994) Nucl. Acids Res., 22, 5767-5768), for the expression in insect cells, e.g., Baculovirus-vectors as e.g. described in EP-B1-0 127 839 or EP-B1-0 549 721, and for the expression in mammalian cells, e.g., the vectors Rc/CMV and Rc/RSV or SV40-vectors, which are commonly known and commercially available.

The molecular biological methods for the production of these expression vectors, as well as the methods of transfecting host cells and culturing such transfected host cells as well as the conditions for producing and obtaining the polypeptides of the invention from said transformed host cells are well known to the skilled person.

The present invention further relates to a host cell comprising a nucleic acid of the invention and/or a vector of the invention, particularly wherein the host cell is a microorganism like yeast or other fungi, like *Escherichia coli, Bacillus subtilis* or other bacteria. The host cell can also be a cell of higher eucaryotic origin, like an insect cell, preferably a virus infected insect cell, more preferably a baculovirus infected insect cell, or like a mammalian cell like HeLa, COS, MDCK 293-EBNA1, NS0 or a hybridoma cell.

The present invention relates further to a method for the production of a polypeptide of the invention, particularly an antibody fragment of the invention, comprising culturing a microorganism transformed with a recombinant vector comprising DNA encoding a polypeptide of the invention, particularly an antibody fragment of the invention, and recovering said a polypeptide of the invention, particularly an antibody fragment of the invention or a fusion protein containing it, from the medium.

The present invention shows that blocking MCAM function may inhibit invasiveness of certain cancer cells derived from group selected of cancer cell-types of pages 7 and 8, and particularly inhibits invasiveness of selected cancer cells, e.g., derived from human sarcoma cells, epithelial tumors, mesenchymal tumors, reticuloendothelial tumors, nervous system tumors, teratomas, even more preferrably human sarcoma cells.

One embodiment of the invention is therefore the use of at least one, particularly one, molecule inhibiting MCAM function in the manufacture of a medicament for the treatment or prevention of invasion and/or metastasis of naturally occurring cancer cells, wherein invasiveness and/or metastatic potential of said cancer cells depends on MCAM function.

In one particular embodiment of the invention the molecule inhibits gene expression of MCAM. Particularly, the molecule inhibiting gene expression of MCAM can be an antisense oligonucleotide targeted against MCAM, an interfering double stranded RNA, commonly known as siRNA (small interfering RNA) targeted against MCAM, a siRNA-like hairpin RNAs targeted against MCAM function as inhibitors of gene expression, or a vector leading to cellular presence of siRNA targeted against MCAM or siRNA-like hairpin RNAs targeted against MCAM.

Antisense oligonucleotides are well known in the art as means to reduce gene expression of a specific gene (see, for example, Probst J.C. (2000) Methods 22(3): 271-281; Heasman J. (2002) Dev. Biol. 243(2): 209-214; Castanotto et al. (2000) Methods Enzymol. 313: 401-420 and Jen and Gewirtz (2000) Stem Cells. 18(5): 307-319).

Recently it has been found that short, double stranded RNAs potently and specifically inhibit expression of a mRNA with a complementary exonic sequence. This phenomenon has been termed RNA interference (RNAi). These double stranded RNAs have been termed siRNAs, for small interfering RNAs, have a paired region of at least 18, preferably at least 19 nucleotides (this paired, double stranded region targets them against an mRNA with a complementary exonic sequence), and have a length of 20 nucleotides to 25 nucleotides, preferably of 21 nucleotides to 23 nucleotides (Elbashir et al. (2001) Nature 411 (6836): 428-429). These siRNAs can be synthesized chemically by methods well known in the art and are commercially available (see e.g. suppliers given in Elbashir et al. above and also in Elbashir et al., (2002) Methods 26: 199-213) or by T7 transcription off a suitable DNA template (see Yu et al. (2002) PNAS 99(9): 6047-6052). They can be delivered to a wide range of cell types, in which inhibition of gene expression of a certain gene is desired, by, e.g., synthesizing the two RNA strands, annealing them and transfecting them (see Elbashir et al. and Yu et al., above). Detailed protocols for the application of siRNAs are described in Elbashir et al., (2002) Methods 26: 199-213.

Another way of effecting RNA interference is to transfect a plasmid that leads to the cellular production of siRNAs or siRNA like hairpin RNAs, which are also functional in RNAi. This can be done by plasmids which carry both, the sense and the antisense strand of the siRNA under the control of a mammalian, preferrably human or mouse, U6 promotor, which leads to the transcription of both strands in a transfected cell, some of which anneal to form functional double stranded siRNAs within the transfected cell (Miyagishi and Taira (2002) Nature Biotech. 19: 497-500). Alternatively, the RNA species transcribed from the U6 promotor can be siRNA-like hairpin RNAs which consist of a 19-base pair siRNA stem with the two strands joined by a structured loop and a U₁₋₄ 3' overhang at the end of the antisense strand (see Paul et al. (2002) Nature Biotech. 19: 505-508). Alternatively the RNA species transcribed from the U6 promotor can be short hairpin RNAs, which are transcribed as small temporal RNAs, short hairpin precursors of about 70 nucleotides, and processed into active siRNAs within the cell in which they are transcribed (see Paddison et al. (2002) Genes and Development 16: 948-958). All these methods based on transfection of plasmids have in common that they lead to the cellular production of siRNAs, which leads to the inhibition of the gene with an exonic region complementary to the at least 18 nucleotide long base paired region of the siRNA. It is clear that also future ways to affect the cellular presence of siRNAs will lead to the desired effect of inhibiting MCAM function and are within the scope of the invention.

In another preferred embodiment the molecule inhibiting MCAM inhibits the function of expressed MCAM.

Expressed MCAM is to be understood in this context as MCAM protein already present on naturally occurring cancer cells before any kind of treatment is initiated.

These molecules are particularly molecules, which bind to the extracellular region of MCAM.

The extracellular region of MCAM is defined as that part of the MCAM protein outside of the cellular membrane, particularly the extracellular amino acid loops between amino acid 24 and amino acid 553. It should be appreciated that MCAM is a glycoprotein, so not only the mentioned amino acids, but also the sugar modifications on them are considered as being part of the extracellular region of MCAM.

More particularly this molecule is selected from the group consisting of a small chemical compound, an antibody against MCAM, an antibody fragment against MCAM, a polypeptide of the invention, an anti-idiotypic antibody of the invention and/or a bioconjugate of the invention, especially wherein the molecule is a polypeptide and/or a bioconjugate of the invention.

In another preferred embodiment the naturally occurring cancer cells, which depend on MCAM function for invasiveness and/or metastatic potential can be any cancer cells selected from the group consisting of the tissues or cancer cell-types of pages 7 and 8, particularly they can be of a tissue origin selected from the group consisting of the lung, liver, kidney, stomach, small intestine, bone, spleen, brain, peripheral nervous system, thyroid, pancreatic, endometrial, ovarian, cervical, skin, colon or lymphoid tissue, more particularly the cancer cells are sarcoma cells.

The invention further pertains to the MCAM antigen as a druggable target. Another aspect of the present invention pertains to antibody fragments that bind to human MCAM with high neutralizing capacity.

In another embodiment of the invention, at least one polypeptide of the invention and/or a bioconjugate of the invention are used for identifying additional molecules that specifically bind human MCAM, particularly in screening assays. These methods entail contacting a reference anti-MCAM antibody fragment with a target species comprising the MCAM domain in the presence of a putative competitor test-binding agent. This step of contacting is conducted under conditions suitable for complex formation between the reference antibody fragment and the target species in the absence of the test-binding agent. Complex formation between the reference antibody fragment and the target species in the presence of the test-binding agent is detected as an indicator of specific binding activity of the test-binding agent to MCAM. This screening method is useful for high throughput screening of, e.g., other antibody libraries or antibody fragment libraries, antisense oligonucleotide libraries or peptide and small molecule libraries to identify and characterize additional "molecules binding specifically to MCAM". Competition is determined by an assay in which the antibody fragment, or other binding agent under test substantially inhibits specific binding of the reference antibody fragment to the target species containing the MCAM domain. This can be determined for example by measuring binding of the reference antibody fragment to a target species comprising MCAM domain in the presence and absence of a putative competitor, i.e. a "molecule binding specifically to MCAM " under conditions suitable for complex formation. Numerous types of competitive binding assays are known and routinely practicable within the invention, as described for example in U.S. Pat. No. 4,376,110. Typically, such assays involve the use of a target species containing the MCAM domain (e.g., purified MCAM or a cell line expressing the MCAM antigen), an unlabeled "molecule binding specifically to MCAM ", and a labeled reference antibody fragment or other binding agent. Competitive inhibition is measured by determining the amount of label bound to the target species in the presence of the "molecule binding specifically to MCAM ". Usually the " molecule binding specifically to MCAM " is present in excess. "Molecules binding specifically to MCAM " identified by these competition assays ("competitive binding agents") include antibodies, antibody fragments, peptides, antisense oligonucleotides, small molecules and other binding agents that bind to an epitope or binding site bound by the reference antibody fragment, as well as a "molecule binding specifically to MCAM " that bind to an epitope or binding site sufficiently proximal to an epitope bound by the reference antibody fragment. Preferably, competitive binding agents of the invention will, when present in excess, inhibit specific binding of a reference antibody fragment to a selected target species by at least 10%, preferably by at least 25%, more preferably by at least 50%, and even more preferably by at least 75%-90% or greater.

In addition to a polypeptide of the invention, particularly an antibody fragment or an antibody of the invention, natural or artificial ligands, peptides, anti-sense, or other small molecules capable of specifically targeting human MCAM may be employed. Drugs can be designed to bind or otherwise interact and inhibit human MCAM based upon the present invention. In this regard, rational drug design techniques such as X-ray crystallography, computer-aided (or assisted) molecular modeling (CAMM), quantitative or qualitative structure-activity relationship (QSAR), and similar technologies can be utilized to focus drug discovery efforts. Rational design allows prediction of molecules, which can interact with proteins or specific parts thereof. Such molecule structures can be synthesized chemically and/or expressed in biological systems. Small molecules may be produced by synthesizing organic compounds according to methods that are well known in the art. A plurality of peptides, semi-peptidic compounds or non-peptidic, and organic compounds may be synthesized and then screened in order to find compounds, which bind to MCAM with high neutralizing capacity. Particularly compounds that inhibit MCAM related invasion. See generally Scott and Smith, "Searching for Peptide Ligands with an Epitope Library", Science (1990), 249, pp. 386-90 and Devlin et al., "Random Peptide Libraries: A Source of Specific Protein Binding Molecules", Science, (1990), 249, pp. 40407.

The present invention also provides methods of using the antibody or antibody fragments to inhibit human MCAM activity or to detect human MCAM in sarcoma cells, either in vitro or in vivo. In a preferred embodiment, treating cells expressing the antigen with one or more antibody fragments causes or leads to a reduction or inhibition of the invasive ability of human sarcoma cells.

The migration of tumor cells into tissue is an important step in metastasis. The processes of adhesion and invasion can be studied in the transendothelial model (See, Woodward et al. (2002) Invest Ophthalmol Vis Sci 43, 1708-14 and Vachula et al. (1992) Invasion Metastasis 12, 66-81). The transendothelial model provides a useful *in vitro* system for the investigations of cellular interactions during the invasion process.

The present invention therefore further provides an *in vitro* method to determine the dependency of the invasiveness of a naturally occurring invasive cancer cell on the functionality of MCAM. This method comprises the steps of:
a) contacting the cells with a molecule inhibiting MCAM function;
b) contacting the cancer cell with a gel-like matrix, under conditions suitable for the growth of said cancer cells; and
c) determining the migration of said cancer cells through the gel-forming matrix.

The term "gel-like matrix" as used herein is understood to be a semi-solid substance with a water content of at least 90%, which allows cultivation of cancer cells in contact with the matrix and allows migration of invasive cancer cells through a slab of said "gel-like matrix" of 0,1 mm to 1 mm, preferably 0,3mm thickness, but not migration of non-invasive cells. Examples for such a "gel-like matrix" are substances resembling the extracellular matrix in protein and carbohydrate composition, particularly the commercially available "Matrigel". Particularly the "gel-like matrix" comprises one of the proteins selected from the group consisting of the proteins collagen type IV, fibronectin and laminin. More particularly the gel-like matrix comprises the proteins collagen type IV, fibronectin and laminin. More preferable the gel-like matrix comprises the proteins collagen type IV, laminin, entactin, nidogen and heparan sulfate proteoglycans.

In a preferred embodiment the interfering molecule of step a) is a polypeptide that specifically binds to an extracellular epitope of MCAM, particularly a polypeptide of the invention, more particularly an antibody or an antibody fragment of the invention, still more particularly an antibody fragment, even more particularly a scFv, dsFv, Fv, single domain antibody or diabody, especially a scFv, single domain antibody or diabody and even more preferably a scFv.

In another preferred embodiment step a) of said method comprises the use of an antisense oligonucleotide against MCAM, siRNA or siRNA-like hairpin RNA against MCAM or a vector leading to cellular presence of siRNA against MCAM or siRNA-like hairpin RNA against MCAM. Particularly preferred is the use of siRNA or siRNA-like hairpin RNA against MCAM or a vector leading to cellular presence of siRNA against MCAM or siRNA-like hairpin RNA against MCAM.

The present invention further provides a method of identifying an antibody or antibody fragment binding specifically to the extracellular region of MCAM, by screening a naïve antibody fragment phage display library, wherein these antibody fragments or the antibody (see WO 91/17271 for phage display of an antibody) is capable of inhibiting and, in particular, inhibits the invasiveness of sarcoma cells. Said method comprises the steps of:
a) contacting a phage library of antibody fragments with invasive sarcoma cells;
b) isolating said cells;
c) removing phages bound unspecifically to said cells, e.g. by washing said cells with a buffered detergent solution, under conditions where said cells do not lyse;
d) eluting phages bound to said cells; and
e) determining the identity of the antibody or antibody fragment represented by said eluted phages.

The identity of phages representing the antibody or the antibody fragment obtained with step d) can be determined by, e.g., sequencing the DNA encoding the antibody or the antibody fragment, or, in the case of a commercial library with gridded or numbered phages, by determining the grid position or the number of the phage. The grid position or the number then can reveal the identity of the antibody or the antibody fragment represented by the phage.

After step d) the pool of phages is enriched in phages binding to MCAM. Those phages binding to MCAM can finally be identified by numerous methods known in the art. Phages can be separated to form individual clones and the clones of the phages can be probed with labeled MCAM protein, or a labeled part of the MCAM protein, e.g. an at least seven amino acid long peptide of the extracellular region of MCAM. Clones binding to such a probe are identified as MCAM-binders. Phages can also be affinity purified on purified MCAM protein or on recombinant MCAM

Alternatively, the open reading frame encoding the antibody or antibody fragment can be recloned from the whole enriched pool into an expression vector, the antibody or the antibody fragment can then be expressed in clones of another host cell, and the clone of the host cell carrying the expression vector comprising a nucleic acid encoding for the antibody or the antibody fragment binding specifically to MCAM can be identified, e.g. by the method described above for the identification of relevant phage clones, by the method of Examples 2 or 9, or by affinity purification on recombinant MCAM.

A particular advantage of this method is that an antibody or an antibody fragment specific for the accessible part of the extracellular region of MCAM is obtained, since the initial selection step is performed on intact cells, which present the accessible part of the extracellular region of MCAM for binding of the phages.

In a preferred embodiment of the invention, the above method comprises instead of step e) the further steps of:
e) contacting isolated phages with recombinant MCAM;
f) washing said MCAM with a buffered detergent and/or high salt solution; and
g) eluting phages bound to MCAM; and
h) determining the identity of the antibody or antibody fragment represented by said eluted phages.

In certain embodiments, the antibody fragments expressed on the phages comprise an antibody or an antibody fragment selected from the group consisting of scFv, dsFv, Fab', Fab, F(ab')₂, Fv, single domain antibodies (DABs) and diabodies, more particularly selected from the group consisting of scFv, dsFv, Fv, single domain antibody or diabody, still more particularly selected from the group consisting of scFv, single domain antibody or diabody and even more preferably a scFv.

The "detergent" used in steps c) and f) is a detergent solution, preferably buffered, and can be Tween in a concentration of 0.001- 0.5%, particularly 0.01-0.1 %. "High salt" in step f) is a high salt solution, preferably buffered, and has an ionic strength of 10mM-1M, particularly 20-500mM, more particularly 50-350mM, even more preferably 80-250mM. Typical useful anions are, for example, chloride, citrate, phosphate, hydrogen phosphate or borate. Typical useful cations are, for example, sodium, potassium, lithium, calcium or magnesium.

The buffered solution in the above paragraph typically has a pH of 7-8. For example, DMEM or PBS, particularly with 1-20%, more particularly 5-15%, even more preferably about 10% FCS, can be used as buffers.

Isolation of cells with phages bound to them is effected by gentle centrifugation at g values from 200 to 300 for 3 to 20 minutes, particularly 5 to 10 minutes. Elution of bound phages, both to cells and to immobilized MCAM, is effected by a wash with 2-100mM, particularly 4-50mM, more particularly 5-20mM, even more preferably around 10mM Glycine at a pH of from 0 to 2.5, particularly from 1 to 2.5, more particularly from 1.5 to 2.5.

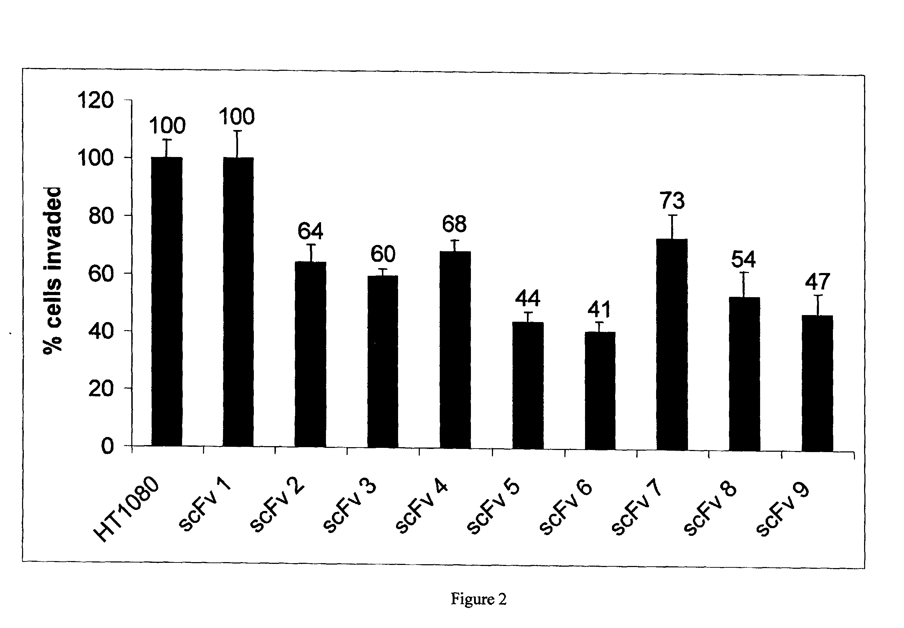

The MCAM inhibitory activity of these antibody fragments, particularly of these scFvs may be assayed as described above, in vivo or in a cell culture experiment. Cell culture assays include assays that determine the inhibitory effect of the antibody fragments of this invention in an invasion or adhesion assay as described in Examples 5 and 7. Results of the invasion assay are provided in Fig 2.

### DESCRIPTION OF THE DRAWINGS

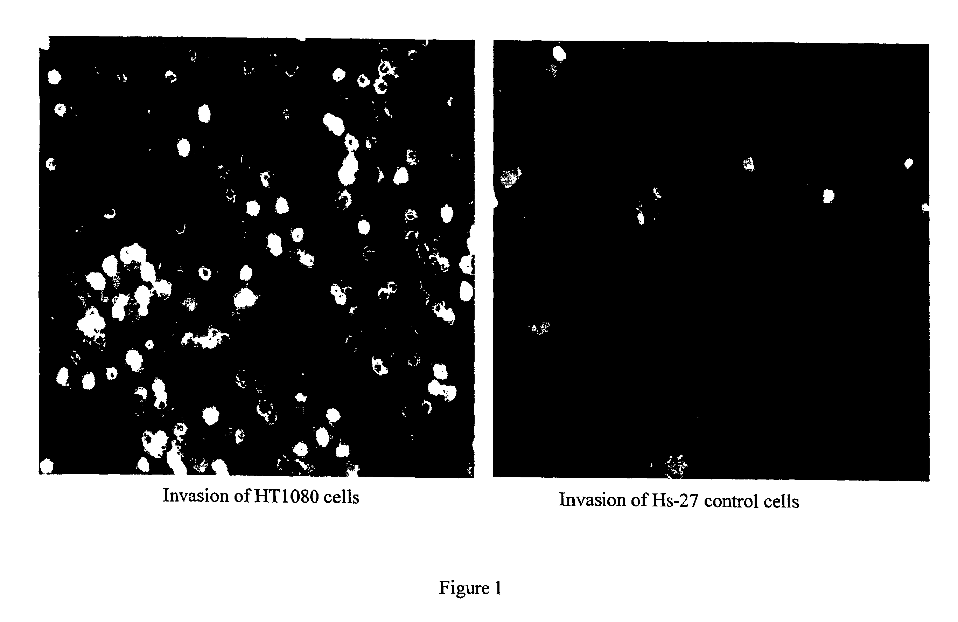

Figure **1** shows invasion of HT1080 stained cells through a 8µm filter. Fluorescence was quantified after six hours incubation at 37°C. Data presented are the mean of n = 3 wells +/- SD.
Figure **2** shows a bar diagram with results of the Invasion Assay
Figure **3** shows results of FACS analysis with HT1080 cells and Hs-27 cells (control cells)

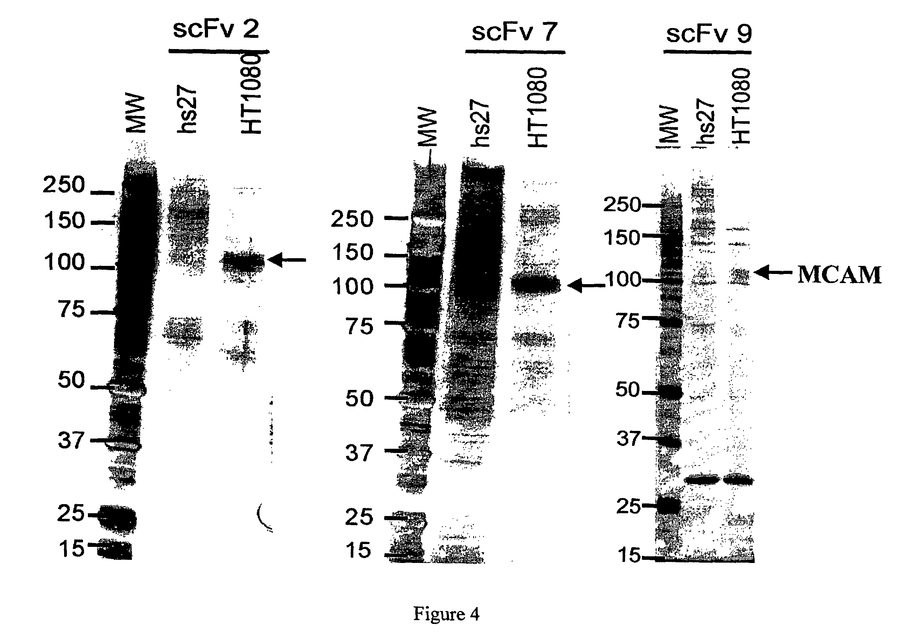

Figure **4** shows results of the immunoprecipitation experiments. The immuno-complexes were separated by SDS-PAGE and silver stained.

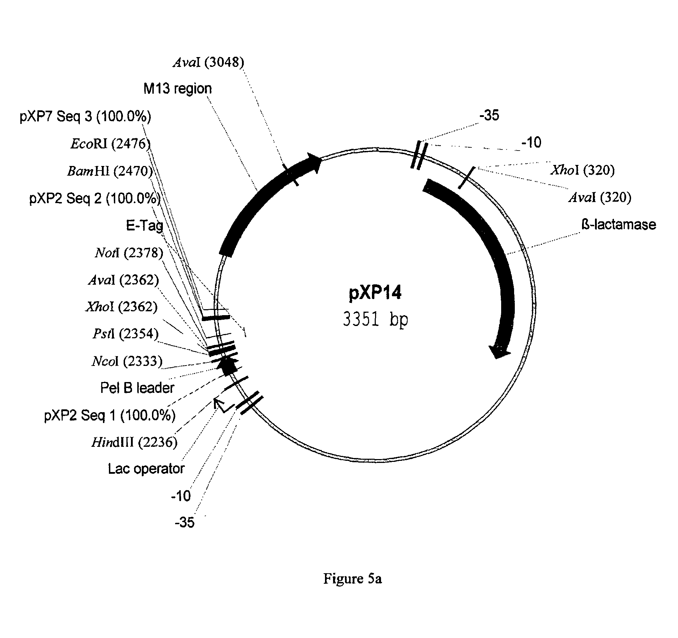

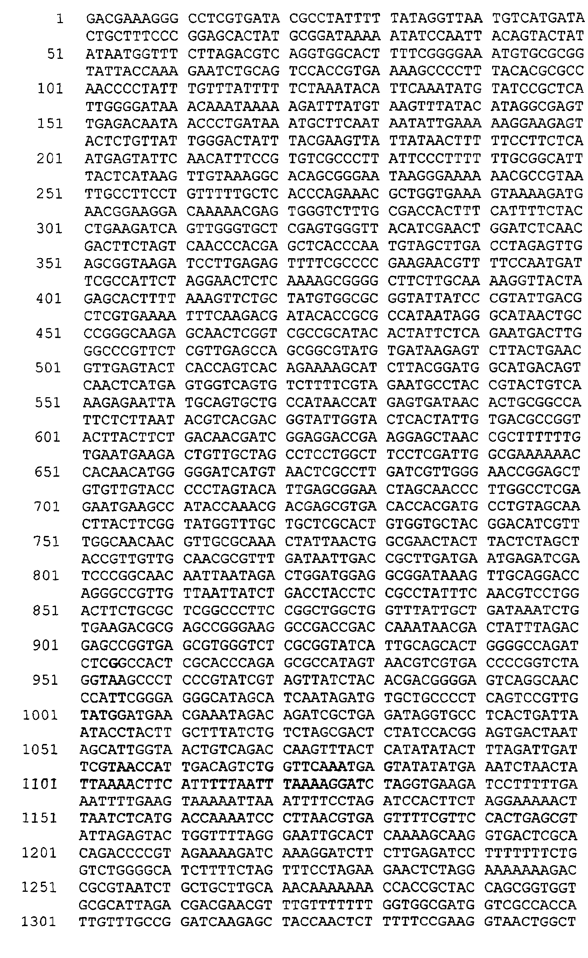

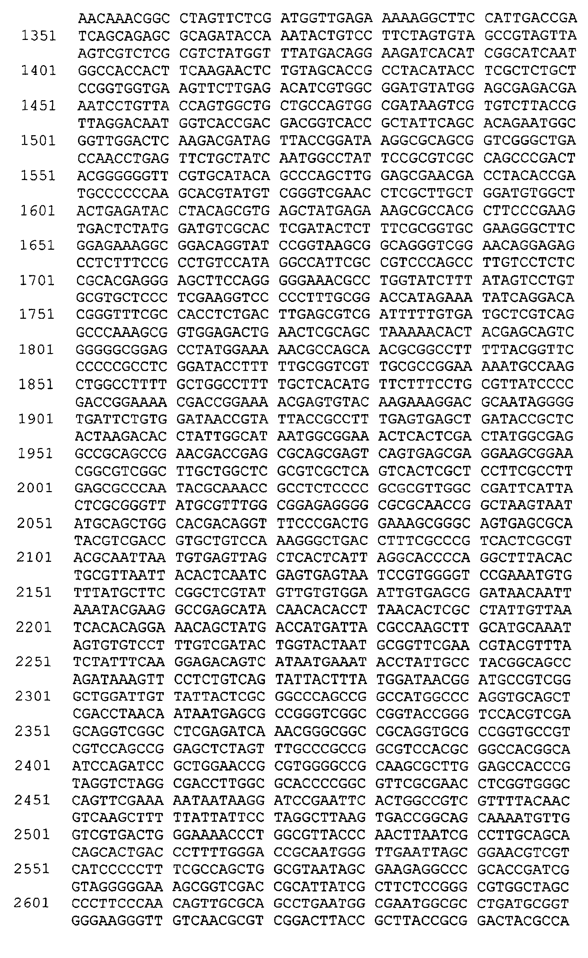

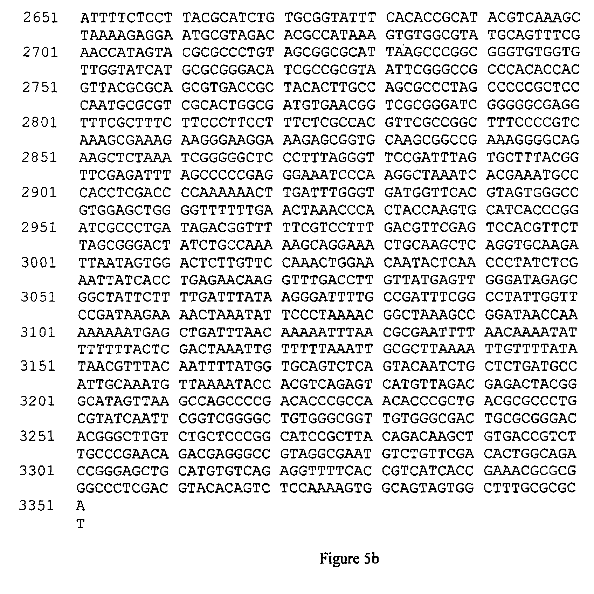

Figure **5a** and **5b** show the vector map and sequence of the scFv expression vector pXP14.

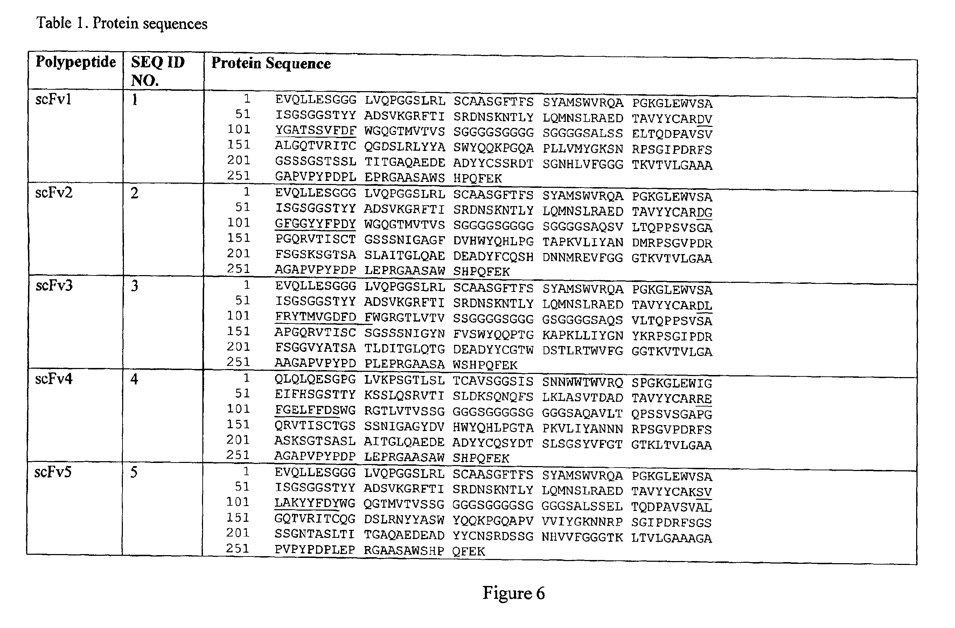

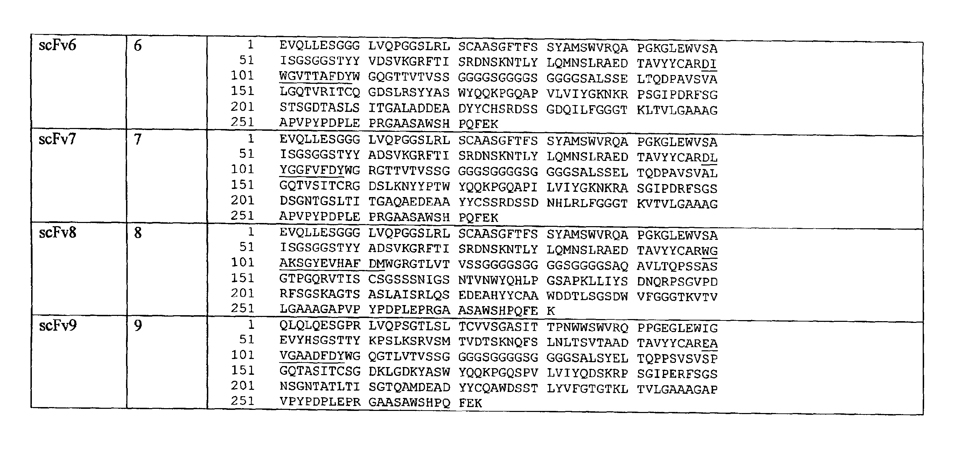

Figure **6** shows Table 1: the peptide sequences of scFv 1 to scFc9.

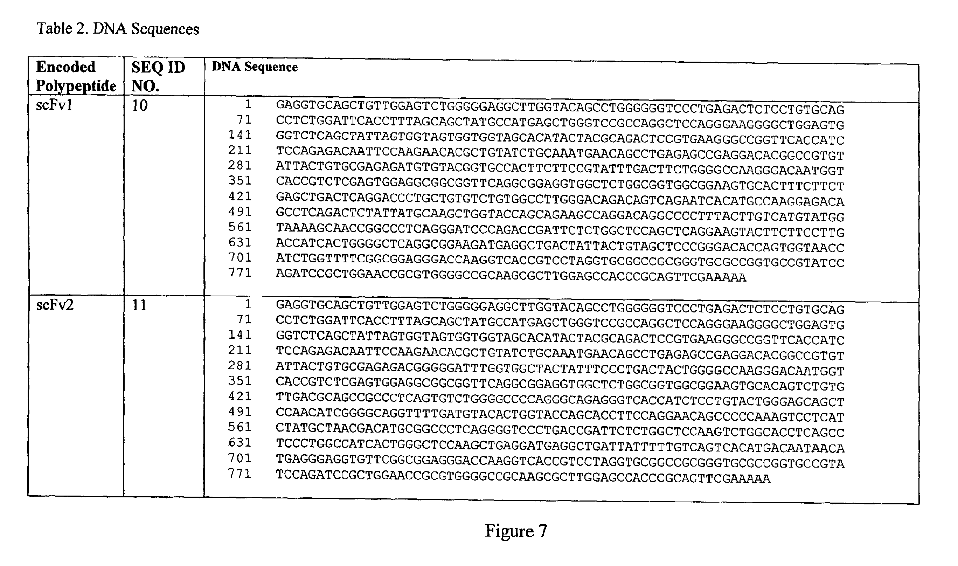

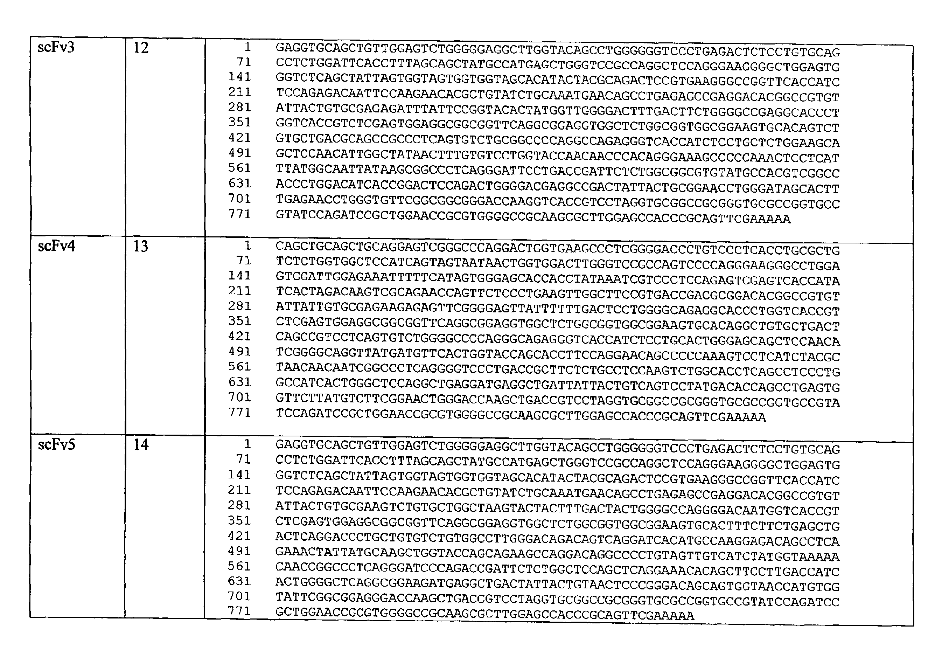

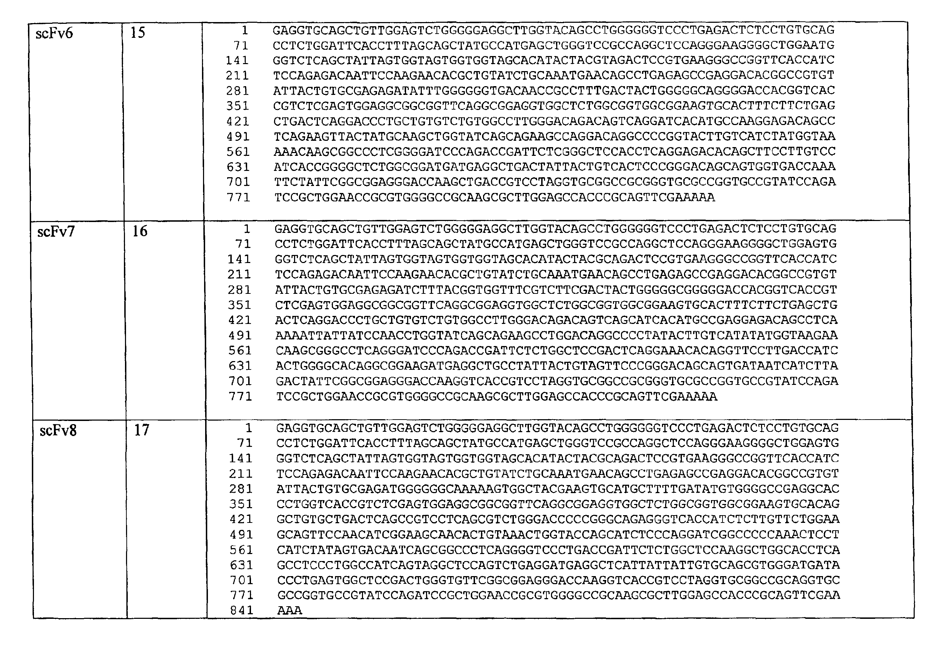

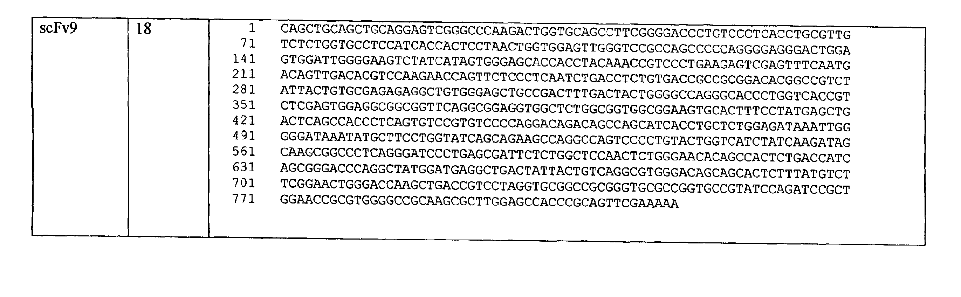

Figure **7** shows Table 2: the nucleotide sequences coding for the polypeptides scFv 1 to scFc9.

The following examples, including the experiments conducted and results achieved, are provided for illustrative purposes only and are not to be construed as limiting upon the present invention.

### EXAMPLES

### Example 1: Selection and screening of scFv (Selection on cells in suspension)

Single chain Fv were selected from a large non-immune phage displayed repertoire of human origin, provided by Cambridge Antibody Technology Ltd., Cambridge, UK.

For selection, HT1080 cells (human fibrosarcoma cell line; ATCC, CCL-121) were harvested with 0.05% EDTA and diluted to 1×10⁷cells/ml in DMEM + 10% FCS. Two times 10¹² cfu of phage library/10⁷ cells were pre-blocked for 1 hour at 25°C with DMEM + 10% FCS and subsequently incubated with end-over-end rotation for 1.5 hour at 25°C with the cells in Eppendorf tubes pre-blocked with DMEM + 10% FCS. Three times 10⁷cells were used for the first round of selection and 1×10⁷ cells were used for the 2^{nd} round of selection, respectively. The cells were washed by centrifugation at 220×g for five minutes, followed by removal of the supernatant and re-suspension in wash buffer. Five washes with DMEM + 10% FCS + 0.05% Tween-20 as wash buffer and five washes with DMEM + 10% FCS as wash buffer were performed. Bound phage were eluted by the addition of 10 mM Glycine pH 2.2, neutralized with 1M Tris/HCl pH 7.4 and used to infect exponentially growing *E. coli* TG1. Phagemid containing *E. coli* were selected and propagated by overnight growth at 30°C on LB agar plates supplemented with 100µg/ml ampicillin and 1% glucose. Phage particles for the 2^{nd} round of selection were produced by super-infecting exponentially growing cultures of the 1^{st} round of selection with helper phage VCS-M13 (Stratagene, La Jolla, CA) and growing the cultures overnight at 20°C in 2×TY supplemented with 100 µg/ml ampicillin and 50 µg/ml kanamycin. Selection ready phage were precipitated with 0.5 M NaCl/4% PEG-6000 from the cleared bacterial supernatant and re-suspended in PBS.

### Example 2: Selection and screening of scFv (Screening on adherent cells)

For screening, the genes encoding the selected scFv, contained in the phage display vector, were re-cloned to the expression vector pXP14. This vector directs the expression of a scFv in fusion with a Streptag and E-tag and does not contain a filamentous phage gene-3. Expression vector containing *E. coli* TG1 from single colonies were grown at 30°C in 2×TY supplemented with 100 µg/ml ampicillin and 0.1% glucose in 96-well microtiter plates (#9297, TPP) until an OD₆₀₀ of 0.7. Expression was induced with IPTG at a final concentration of 0.5 mM and continued at 25°C overnight. Single chain Fv containing cleared lysates were prepared by addition of hen-egg lysozyme (#L-6876, Sigma) to a final concentration of 50 µg/ml for 1 hour at 25°C and centrifugation for 15 minutes at 3000×g. Prior to the screening ELISA, the cleared lysates were blocked by the addition of an equal volume of DMEM + 10% FCS for 1 hour. For the screening ELISA, HT1080 cells were seeded in a 96-well microtiter plate (#9296, TTP) at a density of 3×10⁴ cells/well in DMEM + 10% FCS overnight at 37°C. The wells were blocked with DMEM + 10% FCS for 1 hour at 37°C and the scFv containing blocked cleared lysates added for 1.5 hours at 25°C. The plates were washed 2x with PBS + 0.1% Tween-20 and 1x with PBS, incubated with HRP conjugated α-E-tag (#27-9413-01, Pharmacia Biotech; diluted 1:5000 in 5% Skim Milk Powder (#70166, Fluka) in PBS with 0.1 % Tween-20) for 1 hour, washed 3x with PBS + 0.1% Tween-20 and 3x with PBS, developed with POD (#1 484 281, Roche) and signals read at 370 nm. Positive clones were retested against HT1080 cells and control human fibroblasts Hs-27 (ATCC CRL-1634) using the ELISA screening procedure described above.

### Example 3: Sequencing

Sequencing of scFv genes was performed by Sequiserve GmbH, Vaterstetten, Germany using the primer pXP2 Seq2 (5'-CCCCACGCGGTTCCAGC-3') and pXP2 Seq1 (5'TACCTATTGCCTACGGC-3').. Protein sequences are shown in Table 1 and nucleic acid sequences are shown in Table 2.

### Example 4: FACS analysis for tumor cell specific binding

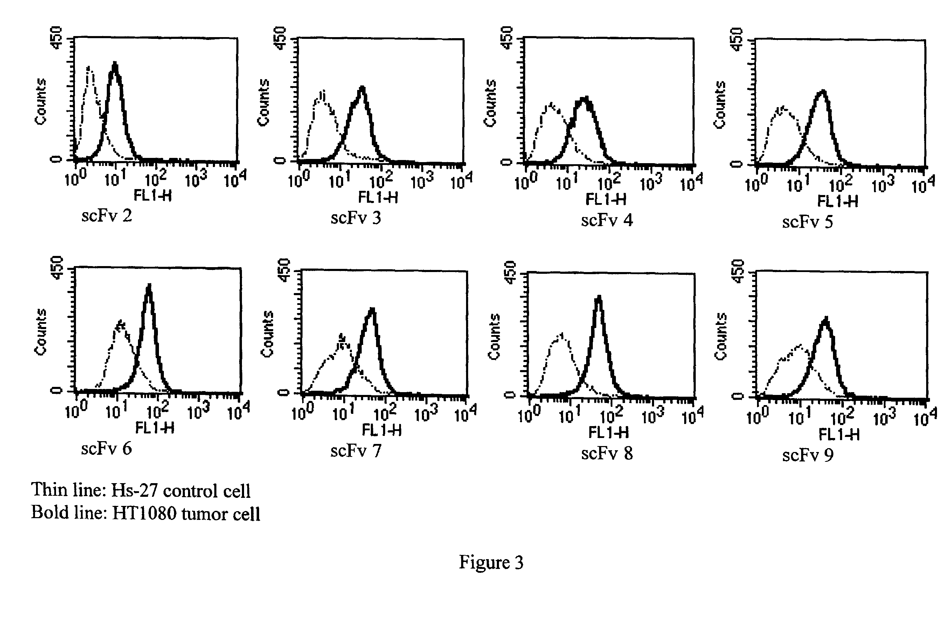

To test the ability of the purified anti HT1080 scFv to bind specifically to the target cells, we performed a fluorescence-activated cell sorter (FACS) analysis using HT1080 cells (10⁶ cells/ml) and Hs-27 cells (10⁶ cells/ml). Cells were incubated with 10µg/ml of pure scFv in CellWash (BD (Becton, Dickinson and Company) #349524) for 20 min at 4°C, washed, and bound scFvs were detected with a secondary FITC labeled anti E-tag mab (Amersham #27-9412-01). Samples were washed and analyzed on a Becton Dickinson FACSscan. Figure 3 shows the log fluorescence intensity (FL1-H; x-axis) versus the relative cell numbers (counts; y-axis) for cells reacting with scFv 2-scFv 9. The thin line represents the control cell line and the bold line the tumor cell line (HT 1080). scFv 1-scFv 9 (data for scFv 1 not shown) specifically stain the tumor cell line with up to 10 fold higher signals compared to the control cell line.

### Example 5: Invasion assay

The ChemoTx® system (Neuro Probe Inc.#106-8, Gaithersburg) was used as a disposable chemotaxis/cell migration chamber in a 96 well format with an 8µm filter Track etched Polycarbonate pore size, 5.7 mm diameter/site.

13,3µl of 0.3mg/ml Matrigel (Matrigel is a solubilized basement membrane preparation extracted from the Engelbreth-Holm-Swarm (EHS) mouse sarcoma, a tumor rich in extracellular matrix proteins. Its major component is laminin, followed by collagen IV, heparan sulfate proteoglycan, entactin and nidogen. It also contains TGF-β fibroblast growth factor, tissue plasminogen activator, and other growth factors which occur naturally in the EHS tumor) (Becton Dickenson, BD #356234) diluted in Dulbeccos PBS (Gibco #14040-091) was applied on the membrane filter of the 96-well plate on row B-H and on row A 1,2 µg/site of collagen S type I (Roche #10982929) diluted in 0,05 M HCl (Sigma #945-50) and incubated over night at 20°C in a desiccator for gelation. HT1080 cells were grown to 70-80% confluence in DMEM supplemented with GlutamaxI (862mg/l (Gibco #31966-021) with 10% FCS (Gibco #10270106). The cells were then labeled *in situ* with Bisbenzimide H 33342 (Sigma #B-2261) diluted 1:100 in DMEM/GlutamaxI/0.1 % BSA (Sigma #A-7030) for 15 min at 37°C, 7,5% CO₂. Cells were washed 2x with DMEM/GlutamaxI/0.1 % BSA and loaded with DMEM/GlutamaxI/0.1 % BSA for 15 min at 37°C, 7,5% CO₂ for recovering. After washing 2x with PBS w/o Ca++, Mg++ (Gibco, 10010-015), the cells were detached with 0.5mM EDTA (Sigma #E8008), collected with Dulbeccos PBS/0.1% BSA/10mM Hepes (Gibco #15630-056), washed 2x with Dulbeccos PBS/0.1% BSA/10mM Hepes, suspended in Dulbeccos PBS/0.1% BSA/10mM Hepes and diluted to 6,7 × 10⁶ cells/ml with Dulbeccos PBS/0.1% BSA/10mM Hepes. 6,7 × 10⁶ cells/ml were incubated 1:1 with 40µg/ml HT1080 specific scFv (1) as a negative control for inhibition of invasion and with HT1080 specific scFv (2-9) for 1h on ice After dilution to 6,7 x 10⁵ cells/ml with DMEM/GlutamaxI/0.1 % BSA, HT1080 cells and HT1080 cell/scFv dilutions were pipetted in triplicate onto the chemotaxis chamber (row B-H) at a density of 3,4 × 10⁴ cells/well and incubated for 6 h at 37°C, 7,5% CO₂. DMEM/GlutamaxI with 5% FCS was used as a chemo attractant in the lower chamber. A standard curve from 1×10⁴ to 4×10⁴ cells/site is performed on collagen S type I coated row A of the chemotaxis chamber. DMEM/GlutamaxI/0.1% BSA was used in the lower chamber (cells are not migrating). After scraping the non-migrating cells from the top of the membrane (except the Standard curve on row A) fluorescence of cells, which had migrated through the membrane (not migrated in case of the Standard curve), was measured on the Fluostar Galaxy (bMG) microplate reader using excitation/emission wavelengths of 370/460nm. In our experiments, a value of 45000 corresponded to 100% migrated cells. Values and error bars in Figure 2 represent the average and standard deviation, respectively, of triplicate samples. Each experiment was repeated three times with essentially similar results.
The invasion phenotype of HT1080 cells was assessed by comparing their relative ability to invade tumor extracellular matrix (Matrigel) using the Transwell culture system described above. The bar diagram (Figure 2) shows the percentage of invasive cells of tumor specific scFvs 1-9. scFv 2-scFv 9 show an inhibitory effect of 26-59% on the invasion of the HT1080 cells. The tumor specific control scFv (scFv 1) has no effect on invasion.

### Example 6: MTS viability assay

Viable cells were detected by measuring the conversion of the tetrazolium dye MTS (MTS, Celltiter A_{queous} one, Promega #G4000) to formazan. HT1080 cells and HT1080 cell/scFv dilutions (obtained from the dilutions prepared in the Invasion assay) were pipetted in triplicate at a density of 3,4 × 10⁴ cells/well were plated in a 96-well plate (black, ultra thin clear flat bottom, special optics, Costar #3615) 10 µl MTS was added to each well and incubated for 1 hour at 37°C, 7,5% CO₂. Absorbance was measured at 492 nm with the Fluostar Galaxy (bMG) microplate reader. For all tested scFvs, no effect on viability of cells was seen (data not shown).

### Example 7: Cell-matrix adhesion assay

96-well plates (TPP #9296) (cell culture treated) were coated with collagen S type I 1µg/well (Roche #10982929) in Dulbeccos PBS (Gibco #14040-091) at 4°C over night. After washing with Dulbeccos PBS, blocking with 2% BSA(Sigma #A-7030)/Dulbeccos PBS for 1h at 37°C and washing with Dulbeccos PBS, HT1080 cells and HT1080 cell/scFv dilutions (obtained from the dilutions prepared in the Invasion assay) were pipetted in triplicate at a density of 3,4 × 10⁴ cells/well and incubated for one hour at 37°C, 7.5% CO₂. After two additional washing steps with Dulbeccos PBS, where non-adherent cells were washed away, a Standard curve from 1×10⁴ to 4×10⁴ stained cells/well (obtained from the dilutions prepared in the Invasion assay) diluted in Dulbeccos PBS/0.1% BSA/10mM Hepes was performed in row A. Washed wells were filled with 50µl Dulbeccos PBS and the absorbance of attached cells and of the Standard curve was measured on the Fluostar Galaxy (bMG) microplate reader using excitation/emission wavelengths of 370/460nm. In our experiment, a value of 25000 corresponded to 100% adherent cells. scFv 1-scFv 9 did not show an inhibitory effect on the adhesion of HT1080 cells on collagen type I (data not shown)

### Example 8: Competition analysis by FACS

To test the ability of the purified anti MCAM scFv to block common MCAM epitopes on the target cells, single cell suspensions of HT1080 were harvested with 0,5mM EDTA/PBS. Approximately 1 × 10⁶ cells were incubated in CellWash (BD, #349524) with 10µg/ml scFv for one hour at 4°C. After washing with Cell Wash 10µg/ml FITC labeled scFv was added and incubated for 20 min at 4°C. Signals of bound FITC labeled scFvs with and without pre incubation of other MCAM binders were analyzed on a Becton Dickinson FACSscan.

### Example 9: Immunoprecipitation

HT1080 and Hs-27 cells (10⁸) were lysed in 3ml 50 mM Tris-HCl, pH 8.0, 150 mM NaCl, 1% Triton X-100 (v/v) containing protease inhibitor cocktail (1 pill in 50ml buffer) (Boehringer Mannheim, Cat.-No. 1697498) and 100 µM Pefablock (Roth, Cat.-No. A154.1). Lysates were pre-incubated for 2h at 4°C with Streptactin Sepharose (IBA, # 2-1201-010) and the supernatants used for the immunoprecipitation reactions. HT1080 specific scFv (50 µg/1 mg cell extract) were added to the cleared lysates, samples rotated for 2h at 4°C, gently centrifuged at 700g to pellet the Streptactin Sepharose, the pellet was washed 4 times with 1ml volume of PBS + 0.1% Tween buffer per wash, before the complexes were isolated by elution from the Streptactin Sepharose pellet with 50 µl 10 mM D-desthiobiotin in PBS 0.1% Tween 20. The immuno-complexes were separated by SDS-PAGE and silver stained for MS analysis.

The scFvs scFv 1-scFv 9 pulled down a protein, detected as a band on SDS-PAGE by silver staining at a molecular weight of ∼110 kDa. Since the immunoprecipitations were performed using cell extract from HT1080 cells and Hs-27 cells as controls it was observed that this particular band was also present in the control samples, but in all cases to a much lower extent indicating that this protein is likely to be overexpressed in the HT1080 cell line. In the case of the scFv CIIIC2 this band was only detected when HT1080 cell extract was used and not with the Hs-27 cells.

### Example 10: Protein identification via mass spectroscopy

The gel bands obtained from immunoprecipitations followed by SDS PAGE were subjected to a tryptic in-gel digest over night at 37 °C. Peptides were extracted with an aqueous and an organic extraction step using 5% formic acid for the first and 45% ACN/5% TFA in the second extraction. One microliter of the obtained peptide mixtures was mixed in a 1:1 ratio with a solution of α-cyano-4-hydroxycinnamic acid (3 mg/ml), co-crystallized on a stainless steel target and analyzed on a MALDI-TOF instrument yielding peptide mass fingerprints (PMF) in a mass range of m/z 800-3000. The obtained PMF were used to search all entries for the species *homo sapiens* in the NCBI and SwissProt databases. 6 to 15 peptides, obtained in different MALDI-TOF-MS experiments, were found to match cell surface glycoprotein MUC18 precursor, with a mass deviation of less than 10 ppm and a maximum protein coverage of 18% (118/646 residues). Multiple entries of the same protein are available in the database, which contain slight deviations in the amino acid sequences.

The molecular weight of any of the melanoma adhesion proteins is around 72 kDa, but since it is to be expected be glycosylated it is not surprising to find the band on the gel around 110 kDa.

### Example 11: Methods for epitope mapping

An epitope mapping may be carried out according to one of the following methods:

### Example 11.1: "Classical" epitope mapping

Defined fragments of the cDNA for the antigen of interest are expressed as recombinant (fusion)proteins and probed in various assays such as Westernblot or ELISA.

### Example 11.2: Phage display technology

The technique of epitope mapping using random peptide phage display libraries was developed to clone small random fragments of the cDNA for the antigen of interest into the phage protein pIII of filamentous phages and display them on the surface of the phage (Fack et al., (1997) J. Immunol. Methods 7, 43-52). Epitope-displaying phages can be captured with antibodies in a procedure called "biopanning". Sequencing of the inserts of the corresponding phages gives some information on the epitopes. This procedure is in principle capable to identify conformational epitopes.

### Example 11.3: Peptide scan technology

It is based on the synthesis of immobilized peptides on activated membranes using the Fmoc chemistry. Amino acid solutions are applied to the activated membrane leading to a peptide bond between the amino-group on the membrane (the membrane is activated with PEG) and the activated carboxy-group of the applied amino acid. After each cycle a specific washing procedure, acetylation, deprotection and monotoring of free amino-groups is performed. In contrast to the in vivo protein-synthesis membrane bound oligo-peptide chains are stepwise synthesized from C- to the N-terminus. Oligo-peptides containing natural as well as modified amino acids can be synthesized up to a length of 20 amino acids. Following synthesis the membranes are equilibrated and unspecific binding sites are blocked. After incubation with the antibody of interest and several washing steps the detection is performed using an HRP-conjugated secondary antibody in combination with the ECL-System. Membranes can be stripped, regenerated, and re-used up to 10 times depending on the antibody. Small overlapping oligo-peptides that ideally cover the complete amino acid sequence of the antigen of interest are synthesized on a solid support. This method allows the identification of linear epitopes on the amino acid level. It also allows rapid mutational studies.

### Example 12: Depletion of cellular MCAM protein by RNAi against MCAM mRNA

The following ribo-oligonucleotides can be purchased from, e.g. Proligo (Hamburg, Germany, www.proligo.com), Pierce Chemical (part of Perbio Science, Rockford, Illinois, www.perbio.com), or another supplier of RNA oligonucleotides:
Oligo 1A (5'-TATGGTGTTGAATCTGTCTTT-3'),
Oligo 2A (5'-AGACAGATTCAACACCATATT-3'),
Oligo 1B (5'-TATGGTGTACAATCTGTCTTT-3'),
Oligo 2B (5'-AGACAGATTGTACACCATATT-3'),
Oligo 3A (5'- GGAGAGGAAGGTGTGGGTGTT-3'),
Oligo 4A (5'- CACCCACACCTTCCTCTCCTT-3')
Oligo 3B (5'- GGAGAGGAACCTGTGGGTGTT-3'),
Oligo 4B (5'- CACCCACAGGTTCCTCTCCTT-3').

The oligonucleotides 1A and 2A anneal to form siRNA directed against the MCAM mRNA, the oligonucleotides 1B and 2B anneal to form a negative control for the pair above, as the nucleotides in bold are mismatches to the MCAM mRNA. The oligonucleotides 3A and 4A anneal to form siRNA directed against the MCAM mRNA, the oligonucleotides 3B and 4B anneal to form a negative control for the 3A/4A-pair, as the nucleotides in bold are mismatches to the MCAM mRNA.

The Oligonucleotide pairs 1A/2A, 1B/2B, 3A/4A, 3B /4A are annealed using Protocol 2 on page 203 of Elbashir et al., Methods (2002) 26: 199-213. When working with RNA special care is used to avoid RNAse contamination of the oligonucleotides. DEPC-treated water is used for preparing buffers. Gloves are always worn to avoid RNAse-contamination by contact with the skin. For detailed instruction on RNA-related work consult, e.g. Chapter 7.3 and 7.4 of the 2^{nd} edition of Sambrook et al. "Molecular Cloning: A Laboratory Manual" (1989), Cold Spring Harbor Laboratory Press.

HT1080 cells are grown to about 90% confluence in DMEM supplement with Glutamax (Gibco #31966-021) with 10% FCS (Gibco #10270106). 24h before transfection with the annealed oligonuceotide pairs described above, the 90% confluent cells out of a 175-ml cell culture flask are harvested, e.g. trypsinized with 10ml trypsin-EDTA solution (Life Technologies), gently centrifuged and resuspended in 10ml DMEM. The cell suspension is diluted 1:10 with fresh DMEM medium with 10% FCS without antibiotics and transferred as 500µl aliquots into each well of a 24-well plate. 24h after seeding the cells a confluency of about 50% is reached.

Separate wells of these cells are then transfected with the annealed oligonucleotide pairs described above according to Protocol 5 on page 207 of Elbashir et al., Methods (2002) 26: 199-213.

After 2 days of incubation according to step 5 of protocol 5 mentioned above, the cells are harvested from the wells and the cells of each well are processed for Western blot, e.g. samples (corresponding to the same number of cells) from a well tranfected without oligonucleotides (untreated cells), a well transfected with the oligonucleotide pair 1A/2A, the pair 1B/2B (negative control 1/2), the pair 3A/4A and the pair 3B/4B (negative control 3/4) are loaded on an acrylamide gel, after the run the proteins are blotted onto, e.g. nitrocellulose, e.g. using a semi-dry blotting apparatus, and the nitrocellulose membranes are probed with anti-MCAM antibodies.

The amount of MCAM protein detected in at least one of the samples 1A/2A or 3A/4A is reduced by more than 75% in comparison to the untreated cells. A significant reduction of the level of MCAM protein is not detected in the negative controls 1B/2B and 3B/4B in comparison to the untreated cells.

### Example 13: Inhibition of invasion by RNAi against MCAM

HT1080 cells are grown to about 90% confluence in DMEM supplement with Glutamax (Gibco #31966-021) with 10% FCS (Gibco #10270106). 24h before transfection with the annealed oligonuceotide pairs described above, the 90% confluent cells out of a 175-ml cell culture flask are trypsinized with 10ml trypsin-EDTA solution (Life Technologies). The cell suspension is diluted 1:10 with fresh DMEM medium with 10% FCS without antibiotics and transferred as 500ul aliquots into each well of a 24-well plate. 24h after seeding the cells a confluency of about 50% is reached.

Separate wells of these cells are then transfected with the annealed oligonucleotide pairs described above according to Protocol 5 on page 207 of Elbashir et al., Methods (2002) 26: 199-213.

After 2 days of incubation according to step 5 of protocol 5 mentioned above, the cells are labeled *in situ* with Bisbenzimide H 33342 (Sigma #B-2261) for 15 min at 37°C, 7,5% CO₂. After washing, the cells are dissociated with 0.5mM EDTA/PBS (Sigma #E8008), washed and suspended in 0.1% BSA (Sigma #A7030)/DMEM. The cells are then diluted to 6,7 x 10⁵ cells/ml with 0.1% BSA/DMEM, HT1080 cells and added onto the chemotaxis chamber as described in Example 5. Invasiveness is then determined as described in Example 5.

The invasiveness measured in at least one of the samples 1A/2A or 3A/4A is reduced by more than 40% in comparison to the untreated cells. A significant reduction of invasiveness is not detected for the negative controls 1B/2B and 3B/4B in comparison to the untreated cells.

While the invention has been described in what is considered to be its preferred embodiments, it is not to be limited to the disclosed embodiments, but on the contrary, is intended to cover various modifications and equivalents included within the spirit and scope of the appended claims, which scope is to be accorded the broadest interpretation so as to encompass all such modifications and equivalents.

## Claims

1. A polypeptide comprising a sequence selected from the group consisting of SEQ ID NO:1, SEQ ID NO:2, SEQ ID NO:3, SEQ ID NO:4, SEQ ID NO:5, SEQ ID NO:6, SEQ ID NO:7, SEQ ID NO:8 and SEQ ID NO: 9.

2. The polypeptide of claim 1, wherein the polypeptide is an antibody or an antibody fragment.

3. The polypeptide of claim 2, wherein the CDR3 region of said antibody or antibody fragment is identical to one of the CDR3 regions shown in Table 1.

4. The polypeptide of claims 2 or 3, wherein said antibody is selected from the group consisting of human IgA, human IgD, human IgE, human IgG and human IgM; in particular human IgG or human IgM, more particularly human IgG1, human IgG2a, human IgG2b, human IgG3, or human IgG4.

5. The polypeptide of any of claims 1 to 4, wherein said polypeptide is labeled with a detectable label; in particular wherein said detectable label is selected from the group consisting of a radioisotope, an enzyme, a fluorophore and a chromophore.

6. A bioconjugate comprising a polypeptide according to any of claims 1 to 5.

7. Use of a polypeptide according to any of claims 1 to 5 and/or a bioconjugate of claim 6 for the detection of the Melanoma Cell Adhesion Molecule (MCAM).

8. A diagnostic kit comprising a polypeptide according to any of claims 1 to 5 and/or a bioconjugate according to claim 6, and a container.

9. A pharmaceutical composition comprising a polypeptide according to any of claims 1 to 5 and/or a bioconjugate according to claim 6, and a pharmaceutical acceptable carrier.

10. An isolated nucleic acid molecule encoding a polypeptide according to any of claims 1 to 5.

11. The isolated nucleic acid molecule of claim 10 comprising any of the nucleic acid sequences of SEQ ID NO:10, SEQ ID NO:11, SEQ ID NO:12, SEQ ID NO:13, SEQ ID NO:14, SEQ ID NO:15, SEQ ID NO:16, SEQ ID NO:17 and SEQ ID NO: 18.

12. A vector comprising a nucleic acid according to claims 10 or 11.

13. A host cell comprising a nucleic acid according to claims 10 or 11 or a vector according to claim 12.

14. Use of a molecule inhibiting MCAM function in the manufacture of a medicament for the treatment or prevention of invasion and/or metastasis of naturally occuring cancer cells, wherein invasiveness and/or metastatic potential of said cancer cells depends on MCAM function.

15. The use of claim 14, wherein the molecule inhibits gene expression of MCAM, in particular wherein the molecule is an antisense oligonucleotide, an siRNA or siRNA-like hairpin RNA, or a vector leading to the cellular presence of an siRNA or siRNA-like hairpin RNA.

16. The use of claim 14 wherein the molecule inhibits the function of expressed MCAM; in particular wherein the molecule binds to the extracellular region of MCAM; more particularly wherein the molecule is selected from the group consisting of a small chemical compound, an antibody, an antibody fragment, an anti-idiotypic antibody, a polypeptide according to any of claims 1 to 5 and a bioconjugate according to claim 6; especially wherein the molecule is a polypeptide according to claims 1 to 5 and/or a bioconjugate according to claim 6.

17. The use of any of claims 14 to 16, wherein the cancer cells are selected from the group consisting of astrocytoma, fibrosarcoma, myxosarcoma, liposarcoma, oligodendroglioma, ependymoma, medulloblastoma, primitive neural ectodermal tumor (PNET), chondrosarcoma, osteogenic sarcoma, pancreatic ductal adenocarcinoma, small and large cell lung adenocarcinomas, chordoma, angiosarcoma, endotheliosarcoma, squamous cell carcinoma, bronchoalveolarcarcinoma, epithelial adenocarcinoma, and liver metastases thereof, lymphangiosarcoma, lymphangioendotheliosarcoma, hepatoma, cholangiocarcinoma, synovioma, mesothelioma, Ewing's tumor, rhabdomyosarcoma, coloncarcinoma, basal cell carcinoma, sweat gland carcinoma, papillary carcinoma, sebaceousgland carcinoma, papillary adenocarcinoma, cystadenocarcinoma, medullary carcinoma, bronchogenic carcinoma, renal cell carcinoma, bileduct carcinoma, choriocarcinoma, seminoma, embryonal carcinoma, Wilms' tumor, testicular tumor, medulloblastoma, craniopharyngiorna, ependymoma, pinealoma, hemangioblastoma, acoustic neurorna, oligodendroglioma, meningioma, neuroblastorna, retinoblastoma, leukemia, multiplemyelorna, Walden-strom's macroglobulinemia, and heavy and light chain disease, and adenocarcinomas of the uterine cervix, uterine and ovarian epithelial carcinomas, transitional, cell carcinoma of the bladder, B and T cell lymphomas (nodular and diffuse) plasmacytoma, acute and chronic leukemias, soft tissue sarcomas and leiomyosarcomas; in particular wherein the cancer cells are sarcoma cells.

18. An ex *vivo* method of determining the dependency of the invasiveness of a naturally occuring invasive cancer cell on the functionality of MCAM, comprising the steps of:
a) contacting the cancer cell with a molecule inhibiting MCAM function;
b) contacting said cancer cell with a gel-like matrix under conditions suitable for the growth of said cancer cells; and
c) determining the migration of said cancer cells through the gel-like matrix.

19. The method of claim 18, wherein step a) comprises contacting the cell with a polypeptide that specifically binds to an extracellular epitope of MCAM, or wherein step a) comprises the use of an antisense oligonucleotide, an siRNA or siRNA-like hairpin RNA, or a vector leading to the cellular presence of an siRNA or siRNA-like hairpin RNA.

20. The method of claims 18 or 19, wherein the gel-like matrix comprises the proteins collagen type IV, fibronectin and laminin.

21. A method of identifying an antibody or an antibody fragment binding specifically to the extracellular region of MCAM, wherein said antibody or antibody fragment is capable of inhibiting invasiveness of sarcoma cells, said method comprising the steps of:
a) contacting a phage library of antibody or antibody fragments with invasive sarcoma cells;
b) isolating said cells;
c) removing phages bound unspecifically and/or not bound to said cells;
d) eluting phages bound to said cells; and optionally
e) determining the identity of the antibody or antibody fragment represented by said eluted phages.

22. The method of claim 21, instead of step e) further comprising the steps of:
e) contacting eluted phages with immobilized MCAM;
f) washing said MCAM with detergent and/or high salt;
g) eluting phages bound to MCAM; and
h) determining the identity of the antibody or antibody fragment represented by said eluted phages.

23. Use of a polypeptide according to claims 1 to 5 and/or a bioconjugate according to claim 6 for identifying molecules that specifically bind human MCAM.

24. A method of treating or preventing metastasis in a patient, said method comprising administering to said patient a polypeptide according to claims 1 to 5 or a bioconjugate according to claim 6 in an amount effective to inhibit MCAM mediated invasiveness and/or metastatic potential.

25. The method according to claim 24, wherein said method is used to reduce or inhibit the invasion of cancer cells selected from the group consisting of astrocytoma, fibrosarcoma, myxosarcoma, liposarcoma, oligodendroglioma, ependymoma, medulloblastoma, primitive neural ectodermal tumor (PNET), chondrosarcoma, osteogenic sarcoma, pancreatic ductal adenocarcinoma, small and large cell lung adenocarcinomas, chordoma, angiosarcoma, endotheliosarcoma, squamous cell carcinoma, bronchoalveolarcarcinoma, epithelial adenocarcinoma, and liver metastases thereof, lymphangiosarcoma, lymphangioendotheliosarcoma, hepatoma, cholangiocarcinoma, synovioma, mesothelioma, Ewing's tumor, rhabdomyosarcoma, coloncarcinoma, basal cell carcinoma, sweat gland carcinoma, papillary carcinoma, sebaceousgland carcinoma, papillary adenocarcinoma, cystadenocarcinoma, medullary carcinoma, bronchogenic carcinoma, renal cell carcinoma, bileduct carcinoma, choriocarcinoma, seminoma, embryonal carcinoma, Wilms' tumor, testicular tumor, medulloblastoma, craniopharyngiorna, ependymoma, pinealoma, hemangioblastoma, acoustic neuroma, oligodendroglioma, meningioma, neuroblastorna, retinoblastoma, leukemia, multiplemyelorna, Waldenstrom's macroglobulinemia, and heavy and light chain disease, and adenocarcinomas of the uterine cervix, uterine and ovarian epithelial carcinomas, transitional, cell carcinoma of the bladder, B and T cell lymphomas (nodular and diffuse) plasmacytoma, acute and chronic leukemias, soft tissue sarcomas and leiomyosarcomas; in particular wherein the cancer cells are sarcoma cells.


SEQUENCE LISTING

<110> Xerion Pharmaceuticals AG

<120> MCAM Inhibitors

<130> X38060EP

<140> 02015591.7
<141> 2002-07-02

<160> 18

<170> PatentIn version 3.1

<210> 1
<211> 276
<212> PRT
<213> Artificial Sequence

<220>
<223> scFv1 - polypeptide

<220>
<221> MISC_FEATURE
<222> (99)..(110)
<223> CDR3 region

<400> 1

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys

X38060EP.ST25
85                  90                  95

Ala Arg Asp Val Tyr Gly Ala Thr Ser Ser Val Phe Asp Phe Trp Gly
            100                 105                 110

Gln Gly Thr Met Val Thr Val Ser Ser Gly Gly Gly Gly Ser Gly Gly
        115                 120                 125

Gly Gly Ser Gly Gly Gly Gly Ser Ala Leu Ser Ser Glu Leu Thr Gln
    130                 135                 140

Asp Pro Ala Val Ser Val Ala Leu Gly Gln Thr Val Arg Ile Thr Cys
145                 150                 155                 160

Gln Gly Asp Ser Leu Arg Leu Tyr Tyr Ala Ser Trp Tyr Gln Gln Lys
                165                 170                 175

Pro Gly Gln Ala Pro Leu Leu Val Met Tyr Gly Lys Ser Asn Arg Pro
            180                 185                 190

Ser Gly Ile Pro Asp Arg Phe Ser Gly Ser Ser Ser Gly Ser Thr Ser
        195                 200                 205

Ser Leu Thr Ile Thr Gly Ala Gln Ala Glu Asp Glu Ala Asp Tyr Tyr
    210                 215                 220

Cys Ser Ser Arg Asp Thr Ser Gly Asn His Leu Val Phe Gly Gly Gly
225                 230                 235                 240

Thr Lys Val Thr Val Leu Gly Ala Ala Ala Gly Ala Pro Val Pro Tyr
                245                 250                 255

Pro Asp Pro Leu Glu Pro Arg Gly Ala Ala Ser Ala Trp Ser His Pro
            260                 265                 270

Gln Phe Glu Lys
        275

<210>  2

X38060EP.ST25
<211>  277
<212>  PRT
<213>  Artificial Sequence

<220>
<223>  scFv2 - polypeptide

<220>
<221>  MISC_FEATURE
<222>  (99)..(110)
<223>  CDR3 region


<400>  2

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Ser Tyr
            20                  25                  30


Ala Met Ser Trp Val Arg Gln Ala Pro Gly Lys Gly Leu Glu Trp Val
        35                  40                  45


Ser Ala Ile Ser Gly Ser Gly Gly Ser Thr Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Asp Gly Gly Phe Gly Gly Tyr Tyr Phe Pro Asp Tyr Trp Gly
            100                 105                 110


Gln Gly Thr Met Val Thr Val Ser Ser Gly Gly Gly Gly Ser Gly Gly
        115                 120                 125


Gly Gly Ser Gly Gly Gly Gly Ser Ala Gln Ser Val Leu Thr Gln Pro
    130                 135                 140


Pro Ser Val Ser Gly Ala Pro Gly Gln Arg Val Thr Ile Ser Cys Thr

X38060EP.ST25
145                 150                 155                 160

Gly Ser Ser Ser Asn Ile Gly Ala Gly Phe Asp Val His Trp Tyr Gln
                165                 170                 175

His Leu Pro Gly Thr Ala Pro Lys Val Leu Ile Tyr Ala Asn Asp Met
            180                 185                 190

Arg Pro Ser Gly Val Pro Asp Arg Phe Ser Gly Ser Lys Ser Gly Thr
        195                 200                 205

Ser Ala Ser Leu Ala Ile Thr Gly Leu Gln Ala Glu Asp Glu Ala Asp
    210                 215                 220

Tyr Phe Cys Gln Ser His Asp Asn Asn Met Arg Glu Val Phe Gly Gly
225                 230                 235                 240

Gly Thr Lys Val Thr Val Leu Gly Ala Ala Ala Gly Ala Pro Val Pro
                245                 250                 255

Tyr Pro Asp Pro Leu Glu Pro Arg Gly Ala Ala Ser Ala Trp Ser His
            260                 265                 270

Pro Gln Phe Glu Lys
        275

<210>  3
<211>  278
<212>  PRT
<213>  Artificial Sequence

<220>
<223>  scFv3 - polypeptide

<220>
<221>  MISC_FEATURE
<222>  (99)..(111)
<223>  CDR3 region

<400>  3

Glu Val Gln Leu Leu Glu Ser Gly Gly Gly Leu Val Gln Pro Gly Gly

X38060EP.ST25
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Phe Arg Tyr Thr Met Val Gly Asp Phe Asp Phe Trp
            100                 105                 110

Gly Arg Gly Thr Leu Val Thr Val Ser Ser Gly Gly Gly Gly Ser Gly
        115                 120                 125

Gly Gly Gly Ser Gly Gly Gly Gly Ser Ala Gln Ser Val Leu Thr Gln
    130                 135                 140

Pro Pro Ser Val Ser Ala Ala Pro Gly Gln Arg Val Thr Ile Ser Cys
145                 150                 155                 160

Ser Gly Ser Ser Ser Asn Ile Gly Tyr Asn Phe Val Ser Trp Tyr Gln
                165                 170                 175

Gln Pro Thr Gly Lys Ala Pro Lys Leu Leu Ile Tyr Gly Asn Tyr Lys
            180                 185                 190

Arg Pro Ser Gly Ile Pro Asp Arg Phe Ser Gly Gly Val Tyr Ala Thr
        195                 200                 205

Ser Ala Thr Leu Asp Ile Thr Gly Leu Gln Thr Gly Asp Glu Ala Asp

X38060EP.ST25
210                 215                 220

Tyr Tyr Cys Gly Thr Trp Asp Ser Thr Leu Arg Thr Trp Val Phe Gly
225                 230                 235                 240

Gly Gly Thr Lys Val Thr Val Leu Gly Ala Ala Ala Gly Ala Pro Val
                245                 250                 255

Pro Tyr Pro Asp Pro Leu Glu Pro Arg Gly Ala Ala Ser Ala Trp Ser
            260                 265                 270

His Pro Gln Phe Glu Lys
        275

<210>  4
<211>  277
<212>  PRT
<213>  Artificial Sequence

<220>
<223>  scFv4 - polypeptide

<220>
<221>  MISC_FEATURE
<222>  (99)..(108)
<223>

<220>
<221>  MISC_FEATURE
<222>  (99)..(108)
<223>  CDR3 region

<400>  4

Gln Leu Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Asn
            20                  25                  30

Asn Trp Trp Thr Trp Val Arg Gln Ser Pro Gly Lys Gly Leu Glu Trp
        35                  40                  45

X38060EP.ST25

Ile Gly Glu Ile Phe His Ser Gly Ser Thr Thr Tyr Lys Ser Ser Leu
    50                  55                  60

Gln Ser Arg Val Thr Ile Ser Leu Asp Lys Ser Gln Asn Gln Phe Ser
65                  70                  75                  80

Leu Lys Leu Ala Ser Val Thr Asp Ala Asp Thr Ala Val Tyr Tyr Cys
                85                  90                  95

Ala Arg Arg Glu Phe Gly Glu Leu Phe Phe Asp Ser Trp Gly Arg Gly
            100                 105                 110

Thr Leu Val Thr Val Ser Ser Gly Gly Gly Gly Ser Gly Gly Gly Gly
        115                 120                 125

Ser Gly Gly Gly Gly Ser Ala Gln Ala Val Leu Thr Gln Pro Ser Ser
    130                 135                 140

Val Ser Gly Ala Pro Gly Gln Arg Val Thr Ile Ser Cys Thr Gly Ser
145                 150                 155                 160

Ser Ser Asn Ile Gly Ala Gly Tyr Asp Val His Trp Tyr Gln His Leu
                165                 170                 175

Pro Gly Thr Ala Pro Lys Val Leu Ile Tyr Ala Asn Asn Asn Arg Pro
            180                 185                 190

Ser Gly Val Pro Asp Arg Phe Ser Ala Ser Lys Ser Gly Thr Ser Ala
        195                 200                 205

Ser Leu Ala Ile Thr Gly Leu Gln Ala Glu Asp Glu Ala Asp Tyr Tyr
    210                 215                 220

Cys Gln Ser Tyr Asp Thr Ser Leu Ser Gly Ser Tyr Val Phe Gly Thr
225                 230                 235                 240

Gly Thr Lys Leu Thr Val Leu Gly Ala Ala Ala Gly Ala Pro Val Pro
                245                 250                 255

X38060EP.ST25

Tyr Pro Asp Pro Leu Glu Pro Arg Gly Ala Ala Ser Ala Trp Ser His
            260                 265                 270

Pro Gln Phe Glu Lys
        275

<210>  5
<211>  274
<212>  PRT
<213>  Artificial Sequence

<220>
<223>  scFv5 - polypeptide

<220>
<221>  MISC_FEATURE
<222>  (99)..(108)
<223>  CDR3 region

<400>  5

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Val Leu Ala Lys Tyr Tyr Phe Asp Tyr Trp Gly Gln Gly
            100                 105                 110

X38060EP.ST25

Thr Met Val Thr Val Ser Ser Gly Gly Gly Gly Ser Gly Gly Gly Gly
        115                 120                 125

Ser Gly Gly Gly Gly Ser Ala Leu Ser Ser Glu Leu Thr Gln Asp Pro
    130                 135                 140

Ala Val Ser Val Ala Leu Gly Gln Thr Val Arg Ile Thr Cys Gln Gly
145                 150                 155                 160

Asp Ser Leu Arg Asn Tyr Tyr Ala Ser Trp Tyr Gln Gln Lys Pro Gly
                165                 170                 175

Gln Ala Pro Val Val Val Ile Tyr Gly Lys Asn Asn Arg Pro Ser Gly
            180                 185                 190

Ile Pro Asp Arg Phe Ser Gly Ser Ser Ser Gly Asn Thr Ala Ser Leu
        195                 200                 205

Thr Ile Thr Gly Ala Gln Ala Glu Asp Glu Ala Asp Tyr Tyr Cys Asn
    210                 215                 220

Ser Arg Asp Ser Ser Gly Asn His Val Val Phe Gly Gly Gly Thr Lys
225                 230                 235                 240

Leu Thr Val Leu Gly Ala Ala Ala Gly Ala Pro Val Pro Tyr Pro Asp
                245                 250                 255

Pro Leu Glu Pro Arg Gly Ala Ala Ser Ala Trp Ser His Pro Gln Phe
            260                 265                 270

Glu Lys

<210>  6
<211>  275
<212>  PRT
<213>  Artificial Sequence

<220>
<223>  scFv6 - polypeptide

X38060EP.ST25

<220>
<221>  MISC_FEATURE
<222>  (99)..(109)
<223>  CDR3 region

<400>  6

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Val Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ile Trp Gly Val Thr Thr Ala Phe Asp Tyr Trp Gly Gln
            100                 105                 110

Gly Thr Thr Val Thr Val Ser Ser Gly Gly Gly Gly Ser Gly Gly Gly
        115                 120                 125

Gly Ser Gly Gly Gly Gly Ser Ala Leu Ser Ser Glu Leu Thr Gln Asp
    130                 135                 140

Pro Ala Val Ser Val Ala Leu Gly Gln Thr Val Arg Ile Thr Cys Gln
145                 150                 155                 160

Gly Asp Ser Leu Arg Ser Tyr Tyr Ala Ser Trp Tyr Gln Gln Lys Pro
                165                 170                 175

X38060EP.ST25

Gly Gln Ala Pro Val Leu Val Ile Tyr Gly Lys Asn Lys Arg Pro Ser
            180                 185                 190

Gly Ile Pro Asp Arg Phe Ser Gly Ser Thr Ser Gly Asp Thr Ala Ser
        195                 200                 205

Leu Ser Ile Thr Gly Ala Leu Ala Asp Asp Glu Ala Asp Tyr Tyr Cys
    210                 215                 220

His Ser Arg Asp Ser Ser Gly Asp Gln Ile Leu Phe Gly Gly Gly Thr
225                 230                 235                 240

Lys Leu Thr Val Leu Gly Ala Ala Ala Gly Ala Pro Val Pro Tyr Pro
                245                 250                 255

Asp Pro Leu Glu Pro Arg Gly Ala Ala Ser Ala Trp Ser His Pro Gln
            260                 265                 270

Phe Glu Lys
        275

<210>  7
<211>  275
<212>  PRT
<213>  Artificial Sequence

<220>
<223>  scFv7 - polypeptide

<220>
<221>  MISC_FEATURE
<222>  (99)..(108)
<223>  CDR3 region

<400>  7

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

X38060EP.ST25

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Tyr Gly Gly Phe Val Phe Asp Tyr Trp Gly Arg Gly
            100                 105                 110

Thr Thr Val Thr Val Ser Ser Gly Gly Gly Gly Ser Gly Gly Gly Gly
        115                 120                 125

Ser Gly Gly Gly Gly Ser Ala Leu Ser Ser Glu Leu Thr Gln Asp Pro
    130                 135                 140

Ala Val Ser Val Ala Leu Gly Gln Thr Val Ser Ile Thr Cys Arg Gly
145                 150                 155                 160

Asp Ser Leu Lys Asn Tyr Tyr Pro Thr Trp Tyr Gln Gln Lys Pro Gly
                165                 170                 175

Gln Ala Pro Ile Leu Val Ile Tyr Gly Lys Asn Lys Arg Ala Ser Gly
            180                 185                 190

Ile Pro Asp Arg Phe Ser Gly Ser Asp Ser Gly Asn Thr Gly Ser Leu
        195                 200                 205

Thr Ile Thr Gly Ala Gln Ala Glu Asp Glu Ala Ala Tyr Tyr Cys Ser
    210                 215                 220

Ser Arg Asp Ser Ser Asp Asn His Leu Arg Leu Phe Gly Gly Gly Thr
225                 230                 235                 240

X38060EP.ST25

Lys Val Thr Val Leu Gly Ala Ala Ala Gly Ala Pro Val Pro Tyr Pro
                245                 250                 255

Asp Pro Leu Glu Pro Arg Gly Ala Ala Ser Ala Trp Ser His Pro Gln
            260                 265                 270

Phe Glu Lys
        275

<210>  8
<211>  281
<212>  PRT
<213>  Artificial Sequence

<220>
<223>  scFv8 - polypeptide

<220>
<221>  MISC_FEATURE
<222>  (99)..(112)
<223>  CDR3 region

<400>  8

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

X38060EP.ST25

Ala Arg Trp Gly Ala Lys Ser Gly Tyr Glu Val His Ala Phe Asp Met
            100                 105                 110

Trp Gly Arg Gly Thr Leu Val Thr Val Ser Ser Gly Gly Gly Gly Ser
        115                 120                 125

Gly Gly Gly Gly Ser Gly Gly Gly Gly Ser Ala Gln Ala Val Leu Thr
    130                 135                 140

Gln Pro Ser Ser Ala Ser Gly Thr Pro Gly Gln Arg Val Thr Ile Ser
145                 150                 155                 160

Cys Ser Gly Ser Ser Ser Asn Ile Gly Ser Asn Thr Val Asn Trp Tyr
                165                 170                 175

Gln His Leu Pro Gly Ser Ala Pro Lys Leu Leu Ile Tyr Ser Asp Asn
            180                 185                 190

Gln Arg Pro Ser Gly Val Pro Asp Arg Phe Ser Gly Ser Lys Ala Gly
        195                 200                 205

Thr Ser Ala Ser Leu Ala Ile Ser Arg Leu Gln Ser Glu Asp Glu Ala
    210                 215                 220

His Tyr Tyr Cys Ala Ala Trp Asp Asp Thr Leu Ser Gly Ser Asp Trp
225                 230                 235                 240

Val Phe Gly Gly Gly Thr Lys Val Thr Val Leu Gly Ala Ala Ala Gly
                245                 250                 255

Ala Pro Val Pro Tyr Pro Asp Pro Leu Glu Pro Arg Gly Ala Ala Ser
            260                 265                 270

Ala Trp Ser His Pro Gln Phe Glu Lys
        275                 280

<210>  9
<211>  273
<212>  PRT

X38060EP.ST25
<213>  Artificial Sequence

<220>
<223>  scFv9 - polypeptide

<220>
<221>  MISC_FEATURE
<222>  (99)..(108)
<223>  CDR3 region


<400>  9

Gln Leu Gln Leu Gln Glu Ser Gly Pro Arg Leu Val Gln Pro Ser Gly
1               5                   10                  15

Thr Leu Ser Leu Thr Cys Val Val Ser Gly Ala Ser Ile Thr Thr Pro
            20                  25                  30

Asn Trp Trp Ser Trp Val Arg Gln Pro Pro Gly Glu Gly Leu Glu Trp
        35                  40                  45

Ile Gly Glu Val Tyr His Ser Gly Ser Thr Thr Tyr Lys Pro Ser Leu
    50                  55                  60

Lys Ser Arg Val Ser Met Thr Val Asp Thr Ser Lys Asn Gln Phe Ser
65                  70                  75                  80

Leu Asn Leu Thr Ser Val Thr Ala Ala Asp Thr Ala Val Tyr Tyr Cys
                85                  90                  95

Ala Arg Glu Ala Val Gly Ala Ala Asp Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Ala Leu Ser Tyr Glu Leu Thr Gln Pro Pro
    130                 135                 140

Ser Val Ser Val Ser Pro Gly Gln Thr Ala Ser Ile Thr Cys Ser Gly
145                 150                 155                 160

X38060EP.ST25

Asp Lys Leu Gly Asp Lys Tyr Ala Ser Trp Tyr Gln Gln Lys Pro Gly
                165                 170                 175

Gln Ser Pro Val Leu Val Ile Tyr Gln Asp Ser Lys Arg Pro Ser Gly
            180                 185                 190

Ile Pro Glu Arg Phe Ser Gly Ser Asn Ser Gly Asn Thr Ala Thr Leu
        195                 200                 205

Thr Ile Ser Gly Thr Gln Ala Met Asp Glu Ala Asp Tyr Tyr Cys Gln
    210                 215                 220

Ala Trp Asp Ser Ser Thr Leu Tyr Val Phe Gly Thr Gly Thr Lys Leu
225                 230                 235                 240

Thr Val Leu Gly Ala Ala Ala Gly Ala Pro Val Pro Tyr Pro Asp Pro
                245                 250                 255

Leu Glu Pro Arg Gly Ala Ala Ser Ala Trp Ser His Pro Gln Phe Glu
            260                 265                 270

Lys

<210>  10
<211>  828
<212>  DNA
<213>  Artificial Sequence

<220>
<223>  DNA encoding the scFv1 polypeptide

<400>  10
gaggtgcagc tgttggagtc tgggggaggc ttggtacagc ctggggggtc cctgagactc
    60

tcctgtgcag cctctggatt cacctttagc agctatgcca tgagctgggt ccgccaggct
   120

ccagggaagg ggctggagtg ggtctcagct attagtggta gtggtggtag cacatactac
   180

gcagactccg tgaagggccg gttcaccatc tccagagaca attccaagaa cacgctgtat

X38060EP.ST25
240

ctgcaaatga acagcctgag agccgaggac acggccgtgt attactgtgc gagagatgtg
300

tacggtgcca cttcttccgt atttgacttc tggggccaag ggacaatggt caccgtctcg
360

agtggaggcg gcggttcagg cggaggtggc tctggcggtg gcggaagtgc actttcttct
420

gagctgactc aggaccctgc tgtgtctgtg gccttgggac agacagtcag aatcacatgc
480

caaggagaca gcctcagact ctattatgca agctggtacc agcagaagcc aggacaggcc
540

cctttacttg tcatgtatgg taaaagcaac cggccctcag ggatcccaga ccgattctct
600

ggctccagct caggaagtac ttcttccttg accatcactg gggctcaggc ggaagatgag
660

gctgactatt actgtagctc ccgggacacc agtggtaacc atctggtttt cggcggaggg
720

accaaggtca ccgtcctagg tgcggccgcg ggtgcgccgg tgccgtatcc agatccgctg
780

gaaccgcgtg gggccgcaag cgcttggagc cacccgcagt tcgaaaaa
828

<210> 11
<211> 831
<212> DNA
<213> Artificial Sequence

<220>
<223> DNA encoding the scFv2 polypeptide

<400> 11
gaggtgcagc tgttggagtc tgggggaggc ttggtacagc ctggggggtc cctgagactc
60

tcctgtgcag cctctggatt cacctttagc agctatgcca tgagctgggt ccgccaggct
120

ccagggaagg ggctggagtg ggtctcagct attagtggta gtggtggtag cacatactac
180

gcagactccg tgaagggccg gttcaccatc tccagagaca attccaagaa cacgctgtat

X38060EP.ST25
240

ctgcaaatga acagcctgag agccgaggac acggccgtgt attactgtgc gagagacggg
300

ggatttggtg gctactattt ccctgactac tggggccaag ggacaatggt caccgtctcg
360

agtggaggcg gcggttcagg cggaggtggc tctggcggtg gcggaagtgc acagtctgtg
420

ttgacgcagc cgccctcagt gtctggggcc ccagggcaga gggtcaccat ctcctgtact
480

gggagcagct ccaacatcgg ggcaggtttt gatgtacact ggtaccagca ccttccagga
540

acagccccca aagtcctcat ctatgctaac gacatgcggc cctcaggggt ccctgaccga
600

ttctctggct ccaagtctgg cacctcagcc tccctggcca tcactgggct ccaagctgag
660

gatgaggctg attatttttg tcagtcacat gacaataaca tgagggaggt gttcggcgga
720

gggaccaagg tcaccgtcct aggtgcggcc gcgggtgcgc cggtgccgta tccagatccg
780

ctggaaccgc gtggggccgc aagcgcttgg agccacccgc agttcgaaaa a
831

<210> 12
<211> 834
<212> DNA
<213> Artificial Sequence

<220>
<223> DNA encoding the scFv3 polypeptide

<400> 12
gaggtgcagc tgttggagtc tgggggaggc ttggtacagc ctggggggtc cctgagactc
60

tcctgtgcag cctctggatt cacctttagc agctatgcca tgagctgggt ccgccaggct
120

ccagggaagg ggctggagtg ggtctcagct attagtggta gtggtggtag cacatactac
180

gcagactccg tgaagggccg gttcaccatc tccagagaca attccaagaa cacgctgtat

X38060EP.ST25
240

ctgcaaatga acagcctgag agccgaggac acggccgtgt attactgtgc gagagattta
300

ttccggtaca ctatggttgg ggactttgac ttctggggcc gaggcaccct ggtcaccgtc
360

tcgagtggag gcggcggttc aggcggaggt ggctctggcg gtggcggaag tgcacagtct
420

gtgctgacgc agccgccctc agtgtctgcg gccccaggcc agagggtcac catctcctgc
480

tctggaagca gctccaacat tggctataac tttgtgtcct ggtaccaaca acccacaggg
540

aaagccccca aactcctcat ttatggcaat tataagcggc cctcagggat tcctgaccga
600

ttctctggcg gcgtgtatgc cacgtcggcc accctggaca tcaccggact ccagactggg
660

gacgaggccg actattactg cggaacctgg gatagcactt tgagaacctg ggtgttcggc
720

ggcgggacca aggtcaccgt cctaggtgcg gccgcgggtg cgccggtgcc gtatccagat
780

ccgctggaac cgcgtggggc cgcaagcgct tggagccacc cgcagttcga aaaa
834

<210> 13
<211> 831
<212> DNA
<213> Artificial Sequence

<220>
<223> DNA encoding the scFv4 polypeptide

<400> 13
cagctgcagc tgcaggagtc gggcccagga ctggtgaagc cctcggggac cctgtccctc
60

acctgcgctg tctctggtgg ctccatcagt agtaataact ggtggacttg ggtccgccag
120

tccccaggga agggcctgga gtggattgga gaaatttttc atagtgggag caccacctat
180

aaatcgtccc tccagagtcg agtcaccata tcactagaca agtcgcagaa ccagttctcc

X38060EP.ST25
240

ctgaagttgg cttccgtgac cgacgcggac acggccgtgt attattgtgc gagaagagag
300

ttcggggagt tattttttga ctcctggggc agaggcaccc tggtcaccgt ctcgagtgga
360

ggcggcggtt caggcggagg tggctctggc ggtggcggaa gtgcacaggc tgtgctgact
420

cagccgtcct cagtgtctgg ggccccaggg cagagggtca ccatctcctg cactgggagc
480

agctccaaca tcggggcagg ttatgatgtt cactggtacc agcaccttcc aggaacagcc
540

cccaaagtcc tcatctacgc taacaacaat cggccctcag gggtccctga ccgcttctct
600

gcctccaagt ctggcacctc agcctccctg gccatcactg ggctccaggc tgaggatgag
660

gctgattatt actgtcagtc ctatgacacc agcctgagtg gttcttatgt cttcggaact
720

gggaccaagc tgaccgtcct aggtgcggcc gcgggtgcgc cggtgccgta tccagatccg
780

ctggaaccgc gtggggccgc aagcgcttgg agccacccgc agttcgaaaa a
831

<210> 14
<211> 822
<212> DNA
<213> Artificial Sequence

<220>
<223> DNA encoding the scFv5 polypeptide

<400> 14
gaggtgcagc tgttggagtc tgggggaggc ttggtacagc ctggggggtc cctgagactc
60

tcctgtgcag cctctggatt cacctttagc agctatgcca tgagctgggt ccgccaggct
120

ccagggaagg ggctggagtg ggtctcagct attagtggta gtggtggtag cacatactac
180

gcagactccg tgaagggccg gttcaccatc tccagagaca attccaagaa cacgctgtat

X38060EP.ST25
240

ctgcaaatga acagcctgag agccgaggac acggccgtgt attactgtgc gaagtctgtg
300

ctggctaagt actactttga ctactggggc caggggacaa tggtcaccgt ctcgagtgga
360

ggcggcggtt caggcggagg tggctctggc ggtggcggaa gtgcactttc ttctgagctg
420

actcaggacc ctgctgtgtc tgtggccttg ggacagacag tcaggatcac atgccaagga
480

gacagcctca gaaactatta tgcaagctgg taccagcaga agccaggaca ggcccctgta
540

gttgtcatct atggtaaaaa caaccggccc tcagggatcc cagaccgatt ctctggctcc
600

agctcaggaa acacagcttc cttgaccatc actggggctc aggcggaaga tgaggctgac
660

tattactgta actcccggga cagcagtggt aaccatgtgg tattcggcgg agggaccaag
720

ctgaccgtcc taggtgcggc cgcgggtgcg ccggtgccgt atccagatcc gctggaaccg
780

cgtggggccg caagcgcttg gagccacccg cagttcgaaa aa
822

<210> 15
<211> 825
<212> DNA
<213> Artificial Sequence

<220>
<223> DNA encoding the scFv6 polypeptide

<400> 15
gaggtgcagc tgttggagtc tgggggaggc ttggtacagc ctggggggtc cctgagactc
60

tcctgtgcag cctctggatt cacctttagc agctatgcca tgagctgggt ccgccaggct
120

ccagggaagg ggctggaatg ggtctcagct attagtggta gtggtggtag cacatactac
180

gtagactccg tgaagggccg gttcaccatc tccagagaca attccaagaa cacgctgtat

X38060EP.ST25
240

ctgcaaatga acagcctgag agccgaggac acggccgtgt attactgtgc gagagatatt
300

tgggggtga caaccgcctt tgactactgg ggcaggggga ccacggtcac cgtctcgagt
360

ggaggcggcg gttcaggcgg aggtggctct ggcggtggcg gaagtgcact ttcttctgag
420

ctgactcagg accctgctgt gtctgtggcc ttgggacaga cagtcaggat cacatgccaa
480

ggagacagcc tcagaagtta ctatgcaagc tggtatcagc agaagccagg acaggccccg
540

gtacttgtca tctatggtaa aaacaagcgg ccctcgggga tcccagaccg attctcgggc
600

tccacctcag gagacacagc ttccttgtcc atcaccgggg ctctggcgga tgatgaggct
660

gactattact gtcactcccg ggacagcagt ggtgaccaaa ttctattcgg cggagggacc
720

aagctgaccg tcctaggtgc ggccgcgggt gcgccggtgc cgtatccaga tccgctggaa
780

ccgcgtgggg ccgcaagcgc ttggagccac ccgcagttcg aaaaa
825

<210> 16
<211> 825
<212> DNA
<213> Artificial Sequence

<220>
<223> DNA encoding the scFv7 polypeptide

<400> 16
gaggtgcagc tgttggagtc tgggggaggc ttggtacagc ctggggggtc cctgagactc
60

tcctgtgcag cctctggatt cacctttagc agctatgcca tgagctgggt ccgccaggct
120

ccagggaagg ggctggagtg ggtctcagct attagtggta gtggtggtag cacatactac
180

gcagactccg tgaagggccg gttcaccatc tccagagaca attccaagaa cacgctgtat

X38060EP.ST25
240

ctgcaaatga acagcctgag agccgaggac acggccgtgt attactgtgc gagagatctt
300

tacggtggtt tcgtcttcga ctactggggg cgggggacca cggtcaccgt ctcgagtgga
360

ggcggcggtt caggcggagg tggctctggc ggtggcggaa gtgcactttc ttctgagctg
420

actcaggacc ctgctgtgtc tgtggccttg ggacagacag tcagcatcac atgccgagga
480

gacagcctca aaaattatta tccaacctgg tatcagcaga agcctggaca ggcccctata
540

cttgtcatat atggtaagaa caagcgggcc tcagggatcc cagaccgatt ctctggctcc
600

gactcaggaa acacaggttc cttgaccatc actggggcac aggcggaaga tgaggctgcc
660

tattactgta gttcccggga cagcagtgat aatcatctta gactattcgg cggagggacc
720

aaggtcaccg tcctaggtgc ggccgcgggt gcgccggtgc cgtatccaga tccgctggaa
780

ccgcgtgggg ccgcaagcgc ttggagccac ccgcagttcg aaaaa
825

<210> 17
<211> 843
<212> DNA
<213> Artificial Sequence

<220>
<223> DNA encoding the scFv8 polypeptide

<400> 17
gaggtgcagc tgttggagtc tgggggaggc ttggtacagc ctggggggtc cctgagactc
60

tcctgtgcag cctctggatt cacctttagc agctatgcca tgagctgggt ccgccaggct
120

ccagggaagg ggctggagtg ggtctcagct attagtggta gtggtggtag cacatactac
180

gcagactccg tgaagggccg gttcaccatc tccagagaca attccaagaa cacgctgtat

X38060EP.ST25
240

ctgcaaatga acagcctgag agccgaggac acggccgtgt attactgtgc gagatggggg
300

gcaaaaagtg gctacgaagt gcatgctttt gatatgtggg gccgaggcac cctggtcacc
360

gtctcgagtg gaggcggcgg ttcaggcgga ggtggctctg gcggtggcgg aagtgcacag
420

gctgtgctga ctcagccgtc ctcagcgtct gggacccccg ggcagagggt caccatctct
480

tgttctggaa gcagttccaa catcggaagc aacactgtaa actggtacca gcatctccca
540

ggatcggccc ccaaactcct catctatagt gacaatcagc ggccctcagg ggtccctgac
600

cgattctctg gctccaaggc tggcacctca gcctccctgg ccatcagtag gctccagtct
660

gaggatgagg ctcattatta ttgtgcagcg tgggatgata ccctgagtgg ctccgactgg
720

gtgttcggcg gagggaccaa ggtcaccgtc ctaggtgcgg ccgcaggtgc gccggtgccg
780

tatccagatc cgctggaacc gcgtggggcc gcaagcgctt ggagccaccc gcagttcgaa
840

aaa
843

<210> 18
<211> 819
<212> DNA
<213> Artificial Sequence

<220>
<223> DNA encoding the scFv9 polypeptide

<400> 18
cagctgcagc tgcaggagtc gggcccaaga ctggtgcagc cttcggggac cctgtccctc
60

acctgcgttg tctctggtgc ctccatcacc actcctaact ggtggagttg ggtccgccag
120

cccccagggg agggactgga gtggattggg gaagtctatc atagtgggag caccacctac

X38060EP.ST25
180

aaaccgtccc tgaagagtcg agtttcaatg acagttgaca cgtccaagaa ccagttctcc
240

ctcaatctga cctctgtgac cgccgcggac acggccgtct attactgtgc gagagaggct
300

gtgggagctg ccgactttga ctactggggc cagggcaccc tggtcaccgt ctcgagtgga
360

ggcggcggtt caggcggagg tggctctggc ggtggcggaa gtgcactttc ctatgagctg
420

actcagccac cctcagtgtc cgtgtcccca ggacagacag ccagcatcac ctgctctgga
480

gataaattgg gggataaata tgcttcctgg tatcagcaga agccaggcca gtcccctgta
540

ctggtcatct atcaagatag caagcggccc tcagggatcc ctgagcgatt ctctggctcc
600

aactctggga acacagccac tctgaccatc agcgggaccc aggctatgga tgaggctgac
660

tattactgtc aggcgtggga cagcagcact ctttatgtct tcggaactgg gaccaagctg
720

accgtcctag gtgcggccgc gggtgcgccg gtgccgtatc cagatccgct ggaaccgcgt
780

ggggccgcaa gcgcttggag ccacccgcag ttcgaaaaa
819